(19) Europäisches Patentamt
European Patent Office
Office européen des brevets

(11) Publication number: **0 231 671 B1**

(12) # EUROPEAN PATENT SPECIFICATION

(45) Date of publication of the patent specification: **16.08.90**

(51) Int. Cl.[5]: **C07J 1/00,** C07J 31/00, C07J 41/00, A61K 31/565

(21) Application number: **86402039.1**

(22) Date of filing: **17.09.86**

(54) **Gonatriene derivatives and process for preparing them.**

(30) Priority: **26.12.85 JP 296135/85**
**14.07.86 JP 165235/86**

(43) Date of publication of application:
**12.08.87 Bulletin 87/33**

(45) Publication of the grant of the patent:
**16.08.90 Bulletin 90/33**

(84) Designated Contracting States:
**BE CH DE FR GB IT LI NL SE**

(56) References cited:
**FR-M-7 158**
**GB-A-1 524 917**
**US-A-3 257 278**
**US-A-3 453 267**

(73) Proprietor: **MITSUBISHI KASEI CORPORATION, 5-2, Marunouchi 2-chome Chiyoda-ku, Tokyo 100(JP)**

(72) Inventor: **Nitta, Issei, 13-22 Narusedai 4-chome, Machida-shi Tokyo(JP)**
Inventor: **Ueno, Hiroaki, 18-24-302 Soshigaya 4-chome, Setagaya-Ku Tokyo(JP)**
Inventor: **Hirabayashi, Norio, 6-5 Sakai-machi Kawasaki-Ku, Kawasaki-shi Kanagawa-ken(JP)**

(74) Representative: **Gutmann, Ernest et al, S.C. Ernest Gutmann - Yves Plasseraud 67, boulevard Haussmann, F-75008 Paris(FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 231 671 B1

## Description

This invention relates to novel gonatriene derivatives which have the activities of controlling the production and the release of pituitary gonadotropin, that is, the so-called anti-gonadotropic activities and accordingly are useful as pharmaceutical agents for treating endometriosis. This invention also relates to a process for preparing these gonatriene derivatives.

Danazol has been known to have the anti-gonadotropic activity and be very effective as a pharmaceutical agent for treating endometriosis, while the need for the pharmaceutical agents having the activity higher than that of Danazol has been very strong.

French patent N° 7 158M discloses 17-α-methoxymethyl-17β-hydroxy-13β-methyl-gona-4,9,11-triene-3-one presented as a drug for the treatment of hyperandrogeny.

US patent 3 453 267 discloses a process for the production of 3-oxo-19-nor-$D^{4,9,11}$-trienic steroids presented as useful for the treatment of disturbances of protidic anabolism, asthenia, thinness, osteporosis, senescence, retardation of consolidation of fractures, metabolic disturbances due to prolonged corticotherapy, hypercholesterolemia, said compounds being both preventive or curative for arterial disturbances, etc.

British patent 1 524 917 discloses gona-4,9(10)-dienes presented as able of being advantageously used in pharmaceutical preparations for the treatment of endocrinopathies and for reproduction control in humans and animals.

This invention now provides novel compounds which have higher anti-gonadotropic activities and accordingly can be used in less dose for treating endometriosis as compared with Danazol, as well as a process for preparing these compounds.

The compounds according to this invention are gonatriene derivatives having the general formula (I):

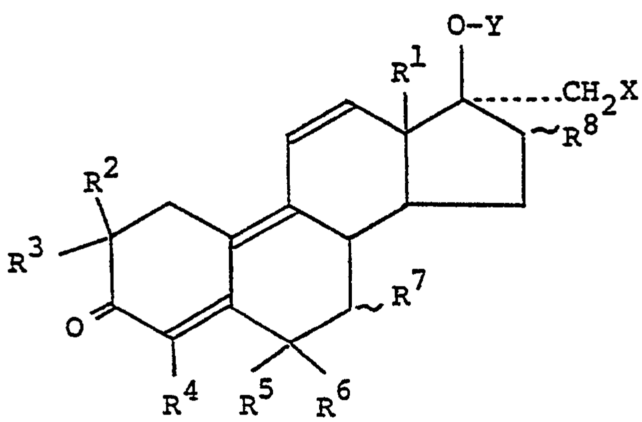

(I)

wherein $R^1$ represents alkyl group having 1 to 3 carbon atoms;
X represents halogen atom, cyano group, $-OR^9$, $-SR^9$, $-SOR^9$ or -SCN where $R^9$ is alkyl group having 1 to 3 carbon atoms;
$R^2$, $R^3$, $R^4$ and $R^7$ independently represent hydrogen atom, halogen atom or alkyl group having 1 to 3 carbon atoms;
$R^5$ and $R^6$ independently represent hydrogen atom, halogen atom, alkyl group having 1 to 3 carbon atoms or hydroxy group, or jointly form oxo group;
$R^8$ represents hydrogen atom or alkyl group having 1 to 3 carbon atoms; and
Y represents hydrogen atoms, $-COR^{10}$ or $-R^{11}$ where each $R^{10}$ and $R^{11}$ are alkyl group or aryl group optionally substituted with halogen atoms, with the proviso that the above-defined groups simultaneously have the following meanings: $R_2$, $R_3$, $R_4$, $R_5$, $R_6$, $R_7$, $R_8$ and Y represents hydrogen atoms, and X represents $-OCH_3$.

The invention also relates to the use of the compounds defined above, as well as of 17α-methoxymethyl-17β-hydroxy-13β-methyl-gona 4,9,11-triene-3-one, for the preparation of pharmaceutical agents for the treatment of endometriosis.

In the above general formula (I), $R^1$ represents alkyl group having 1 to 3 carbon atoms, that is, methyl, ethyl, n-propyl or i-propyl groups and methyl or ethyl group is preferred.

X represents halogen atom, that is, fluorine, chlorine, bromine or iodine atoms and chlorine atom is preferred. Alternatively, X may represent cyano group,

$$-OR^9 \;,\; -SR^9 \;,\; -\overset{\overset{O}{\uparrow}}{S}R^9 \text{ or } -SCN$$

where $R^9$ is alkyl group having 1 to 3 carbon atoms, methyl group being preferred.

Each of $R^2$ and $R^3$ represents hydrogen atom, halogen atom or alkyl group having 1 to 3 carbon atoms and hydrogen atom or methyl group is preferred.

$R^4$ represents hydrogen atom, halogen atom or alkyl group having 1 to 3 carbon atoms and hydrogen atom, chlorine atom or methyl group is preferred.

Each of $R^5$ and $R^6$ represents hydrogen atom, halogen atom, alkyl group having 1 to 3 carbon atoms or hydroxy group and hydrogen atom, chlorine atom, methyl group or hydroxy group is preferred. Alternatively and preferably, $R^5$ and $R^6$ jointly form oxo (=O) group.

$R^7$ represents hydrogen atom, halogen atom or alkyl group having 1 to 3 carbon atoms and hydrogen atom or methyl group is preferred.

$R^8$ represents hydrogen atom or alkyl group having 1 to 3 carbon atoms; preferred are hydrogen, methyl or ethyl.

Y represents a hydrogen atom, $-C{=}OR^{10}$ or $-R^{11}$ where each of $R^{10}$ and $R^{11}$ is alkyl or aryl group optionally substituted with halogen atoms; preferred is the hydrogen atom or the $-COCH_3$ group.

The preferable compounds according to this invention are exemplified as follows.

17α-cyanomethyl-17β-hydroxy-13β-methyl-gona-4,9,11-triene-3-one,
17α-cyanomethyl-17β-hydroxy-13β-ethyl-gona-4,9,11-triene-3-one,
17α-cyanomethyl-17β-hydroxy-13β-propyl-gona-4,9,11-triene-3-one,
17α-cyanomethyl-17β-hydroxy-2α,13β-dimethyl-gona-4,9,11-triene-3-one,
17α-cyanomethyl-17β-hydroxy-2α-methyl-13β-ethyl-gona-4,9,11-triene-3-one,
17α-cyanomethyl-17β-hydroxy-2β,13β-dimethyl-gona-4,9,11-triene-3-one,
17α-cyanomethyl-17β-hydroxy-2β-methyl-13β-ethyl-gona-4,9,11-triene-3-one,
17α-cyanomethyl-17β-hydroxy-2,2,13β-trimethyl-gona-4,9,11-triene-3-one,
17α-cyanomethyl-17β-hydroxy-2,2-dimethyl-13β-ethyl-gona-4,9,11-triene-3-one,
17α-cyanomethyl-17β-hydroxy-4,13β-dimethyl-gona-4,9,11-triene-3-one,
17α-cyanomethyl-17β-hydroxy-4-methyl-13β-ethyl-gona-4,9,11-triene-3-one,
17α-cyanomethyl-17β-hydroxy-4-chloro-13β-methyl-gona-4,9,11-triene-3-one,
17α-cyanomethyl-17β-hydroxy-4-chloro-13β-ethyl-gona-4,9,11-triene-3-one,
17α-cyanomethyl-17β-hydroxy-6,13β-dimethyl-gona-4,9,11-triene-3-one,
17α-cyanomethyl-17β-hydroxy-6-methyl-13β-ethyl-gona-4,9,11-triene-3-one,
17α-cyanomethyl-17β-hydroxy-6-chloro-13β-methyl-gona-4,9,11-triene-3-one,
17α-cyanomethyl-17β-hydroxy-6 chloro-13β-ethyl-gona-4,9,11-triene-3-one,
17α-cyanomethyl-6,17β-dihydroxy-13β-methyl-gona-4,9,11-triene-3-one,
17α-cyanomethyl-6,17β-dihydroxy-13β-ethyl-gona-4,9,11-triene-3-one,
17α-cyanomethyl-17β-hydroxy-7α,13β-dimethyl-gona-4,9,11-triene-3-one,
17α-cyanomethyl-17β-hydroxy-7α-methyl-13β-ethyl-gona-4,9,11-triene-3-one,
17α-cyanomethyl-17β-hydroxy-16,13β-dimethyl-gona-4,9,11-triene-3-one,
17α-cyanomethyl-17β-hydroxy-16-methyl-13β-ethyl-gona-4,9,11-triene-3-one
17α-cyanomethyl-17β-hydroxy-16-ethyl-13β-methyl-gona-4,9,11-triene-3-one,
17α-cyanomethyl-17β-hydroxy-16,13β-diethyl-gona-4,9,11-triene-3-one,
17α-cyanomethyl-17β-hydroxy-16-i-propyl-13β-methyl-gona-4,9,11-triene-3-one,
17α-cyanomethyl-17β-hydroxy-16-i-propyl-13β-ethyl-gona-4,9,11-triene-3-one,
17α-cyanomethyl-17β-hydroxy-13β-methyl-gona-4,9,11-triene-3-one 17β-acetate,
17α-cyanomethyl-17β-hydroxy-13β-ethyl-gona-4,9,11-triene-3-one 17β-acetate,
17α-chloromethyl-17β-hydroxy-13β-methyl-gona-4,9,11-triene-3-one,
17α-chloromethyl-17β-hydroxy-13β-ethyl-gona-4,9,11-triene-3-one,
17α-chloromethyl-17β-hydroxy-13β-propyl-gona-4,9,11-triene-3-one,
17α-fluoromethyl-17β-hydroxy-13β-ethyl-gona-4,9,11-triene-3-one,
17α-bromomethyl-17β-hydroxy-13β-ethyl-gona-4,9,11-triene-3-one,
17α-methoxymethyl-17β-hydroxy-13β-ethyl-gona-4,9,11-triene-3-one,
17α-methoxymethyl-17β-hydroxy-13β-propyl-gona-4,9,11-triene-3-one,
17α-methylthiomethyl-17β-hydroxy-13β-methyl-gona-4,9,11-triene-3-one,
17α-methylthiomethyl-17β-hydroxy-13β-ethyl-gona-4,9,11-triene-3-one,
17α-methylthiomethyl-17β-hydroxy-13β-propyl-gona-4,9,11-triene-3-one,
17α-methylsulfinylmethyl-17β-hydroxy-13β-methyl-gona-4,9,11-triene-3-one,
17α-methylsulfinylmethyl-17β-hydroxy-13β-ethyl-gona-4,9,11-triene-3-one,
17α-methylsulfinylmethyl-17β-hydroxy-13β-propyl-gona-4,9,11-triene-3-one,
17α-thiocyanatomethyl-17β-hydroxy-13β-methyl-gona-4,9,11-triene-3-one,
17α-thiocyanatomethyl-17β-hydroxy-13β-ethyl-gona-4,9,11-triene-3-one,
17α-thiocyanatomethyl-17β-hydroxy-13β-propyl-gona-4,9,11-triene-3-one.

The gonatriene derivatives having the general formula (I) of this invention can be prepared in accordance with one of two processes which will be explained in detail hereinafter.

The first process for preparing the compounds of this invention is based on the following reaction scheme.

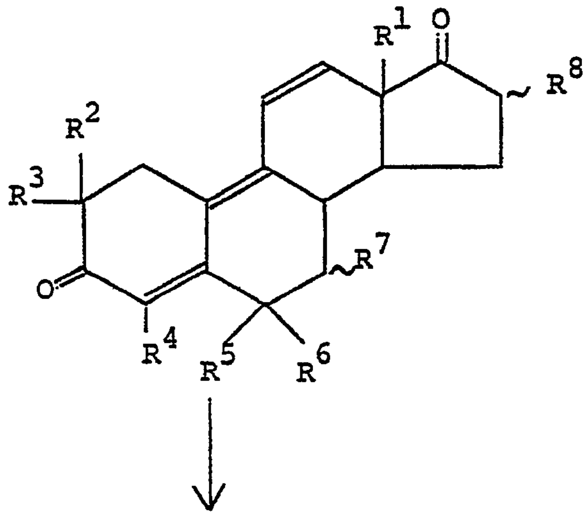

(II)

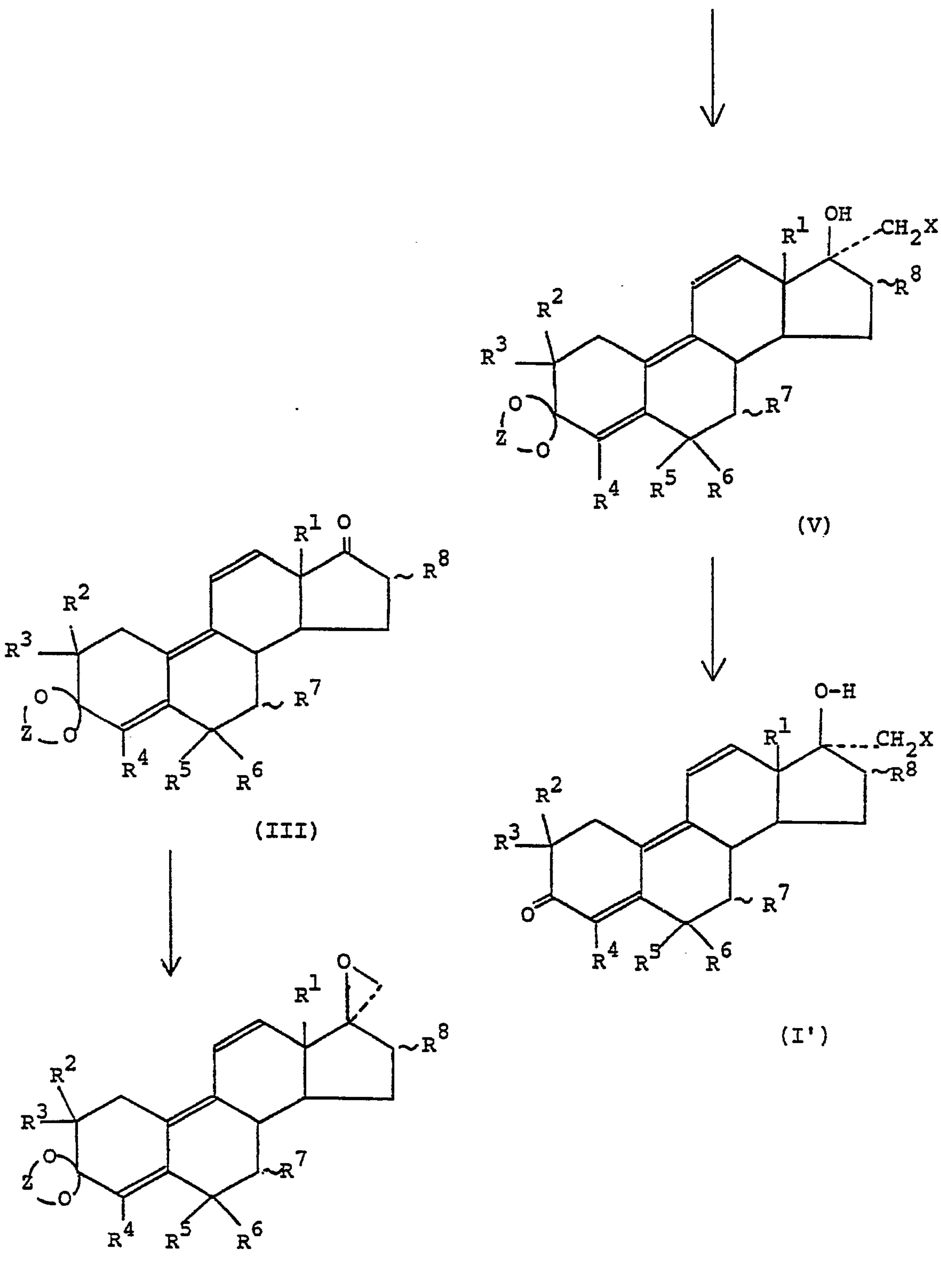

(V)

(III)

(I')

(IV)

The starting material in this first process is diketone having the following formula (II) which is the known compound disclosed in FR 1526961 (1968) and FR 1526962 (1968).

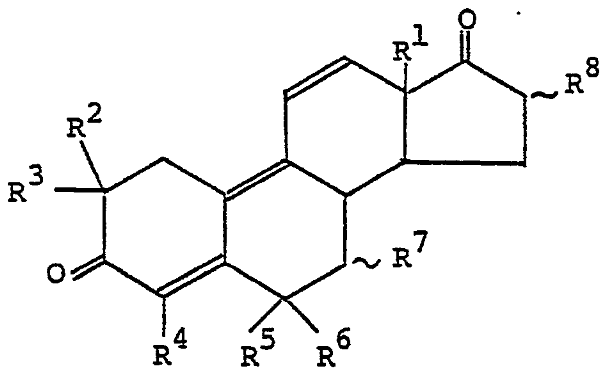

(II)

wherein $R^1$ to $R^8$ are as herein defined.

Diketone (II) is reacted with ethylene glycol, trimethylene glycol or 2,2-dimethyltrimethylene glycol under acidic condition, for example, in the presence of a catalytic amount of an acidic compound such as p-toluene sulfonic acid, sulfuric acid or nitric acid to preferentially protect the carbonyl group in 3 position of the diketone (II) and obtain acetal having the formula (III) :

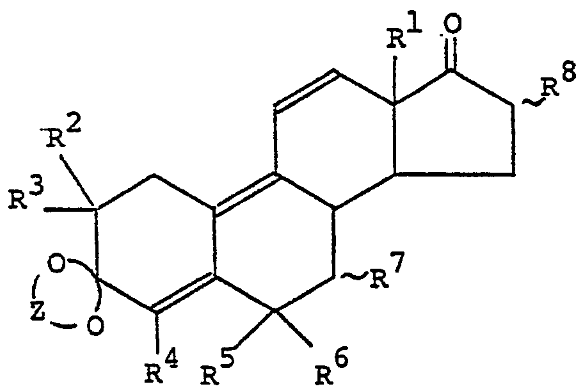

(III)

wherein $R^1$ to $R^8$ are as herein defined and Z is ethylene, trimethylene or 2,2-dimethyltrimethylene groups. For removing the water produced during the reaction, the dehydrating agent such as orthoesters, for example, methyl or ethyl orthoformate is preferably co-exist in this reaction. In place of the use of the dehydrating agent, the azeotropic distillation may be carried out using a solvent capable of forming an azeotropic mixture with the water produced, such as benzene or toluene.

Then, acetal (III) is reacted with dimethylsulfonium methylide or dimethyloxosulfonium methylide in a solvent, preferably an aprotic and high-polar solvent such as dimethyl-sulfoxide, dimethylformamide, dimethylacetamide or N-methyl pyrrolidone, to obtain epoxy triene having the formula (IV) :

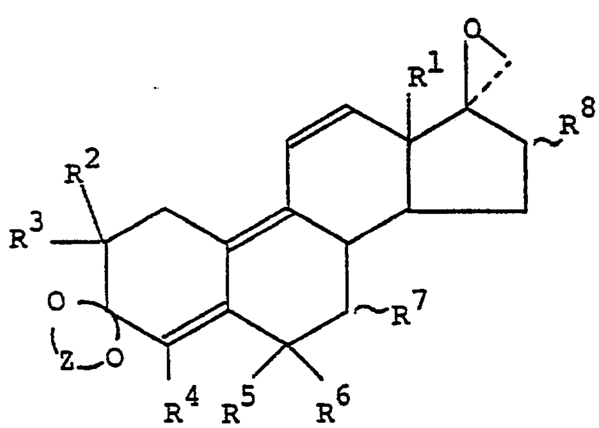

(IV)

wherein $R^1$ to $R^8$ and Z are as herein defined. The reaction temperature is -10 to +50°C, preferably 0 to 30°C. The above-mentioned dimethylsulfonium methylide or dimethyloxosulfonium methylide can be prepared by reacting trimethyl (oxo)sulfonium such as trimethyl(oxo)sulfonium iodide or chloride with a basic

compound such as potassium t-butoxide or sodium hydride. The used amount of trimethyl(oxo)sulfonium is at least 1 mole, preferably 1.2 to 5 moles per mole of acetal (III) and the used amount of the basic compound is at least 1 mole, preferably 1.1 to 1.5 moles per mole of trimethyl(oxo)sulfonium.

Then, epoxy triene (IV) is reacted with cyanide such as potassium or sodium cyanide; halide such as lithium fluoride, chloride, bromide or iodide or potassium iodide; metal alkoxide such as sodium or potassium methoxide, ethoxide or propoxide; metal alkylsulfide such as sodium salt of alkylmercaptan, for example, methyl mercaptan, ethyl mercaptan or propyl mercaptan; or thiocyanate such as sodium thiocyanate in a solvent, preferably alcohol such as methanol, ethanol, propanol or ethylene glycol to obtain the compound of the formula (V) :

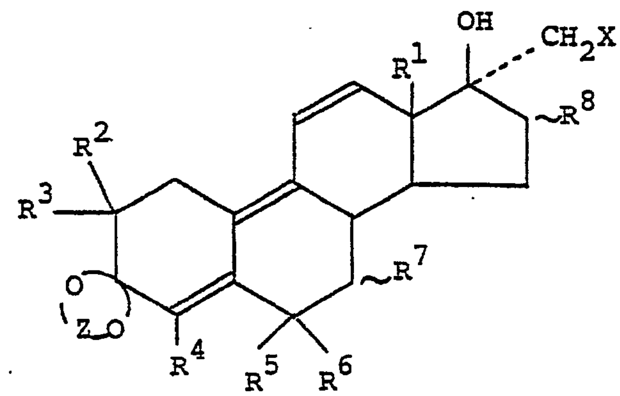

(V)

wherein $R^1$ to $R^8$, X and Z are as herein defined. In the reaction of epoxy triene (IV) with cyanide or halide, it is used in an amount of at least 1 mole, preferably 3 to 10 moles per mole of epoxy triene (IV) and the reaction temperature is more than 0°C, preferably 10 to 50°C. In the reaction of epoxy triene (IV) with metal alkoxide, it is used in an amount of at least 1 mole, preferably 10 to 20 moles per mole of epoxy triene (IV) and the reaction temperature is more than room temperature, preferably refluxing temperature. In the reaction of epoxy triene (IV) with metal alkylsulfide, it is used in an amount of at least 1 mole, preferably 2 to 3 moles per mole of epoxy triene (IV) and the reaction temperature is more than room temperature, preferably 30 to 70°C. In the reaction of epoxy triene (IV) with thiocyanate, generally together with an acetic acid, thiocyanate is used in an amount of at least 1 mole, preferably 10 to 20 moles and acetic acid is used in an amount of at least 1 mole, preferably 5 to 10 moles per mole of epoxy triene (IV) and the reaction temperature is more than room temperature, preferably 30 to 70°C.

Then, the compound (V) is treated in accordance with any of the methods for deprotecting acetal group to obtain gonatriene derivative having the formula (I') :

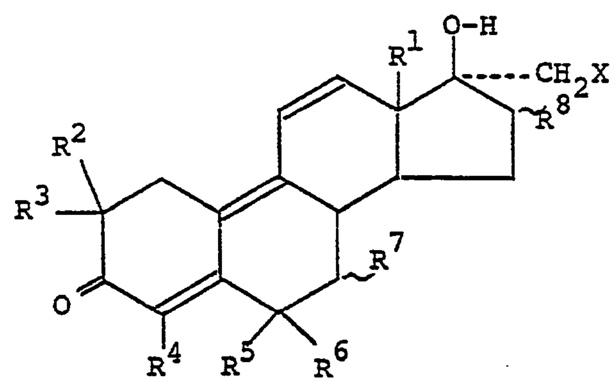

(I')

wherein $R^1$ to $R^8$ and X are as herein defined. The above-mentioned treatment is preferably carried out in a solvent such as ketone, for example, acetone or methyl ethyl ketone in the presence of a catalytic amount of an acidic compound, for example, p-toluene sulfonic acid, sulfuric acid or nitric acid.

Epoxy triene (IV) may be reacted with cyanic acid, hydrogen halide such as hydrochloric acid, hydrobromic acid or hydrofuloric acid or thiocyanic acid in a solvent, preferably dimethylformamide, dimethylsulfoxide, dimethylacetamide, N-methylpyrrolidone, tetrahydrofurane or dioxane to directly obtain gonatriene derivative (I').

The second process for preparing the compounds of this invention is based on the following reaction scheme.

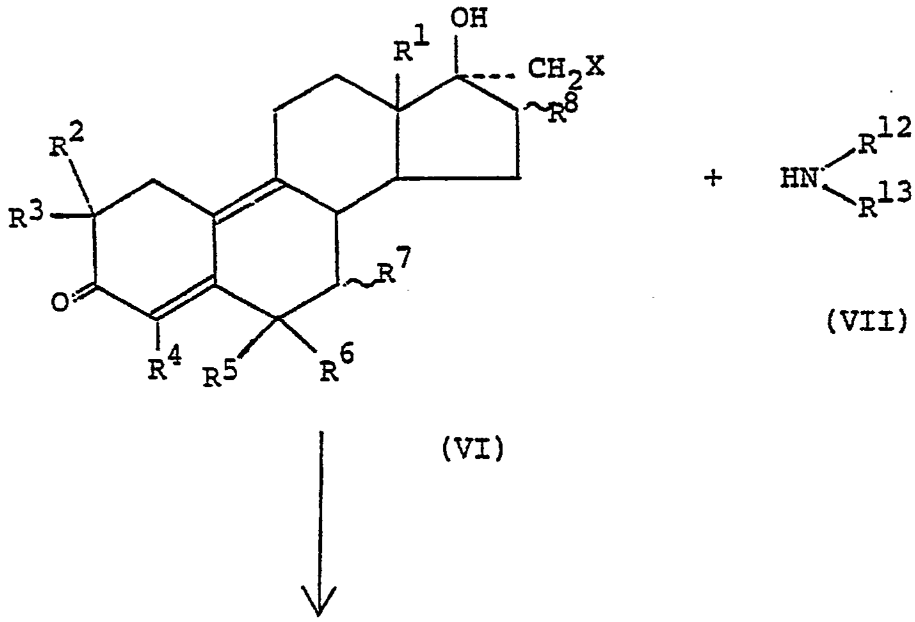

(VI)

(VII)

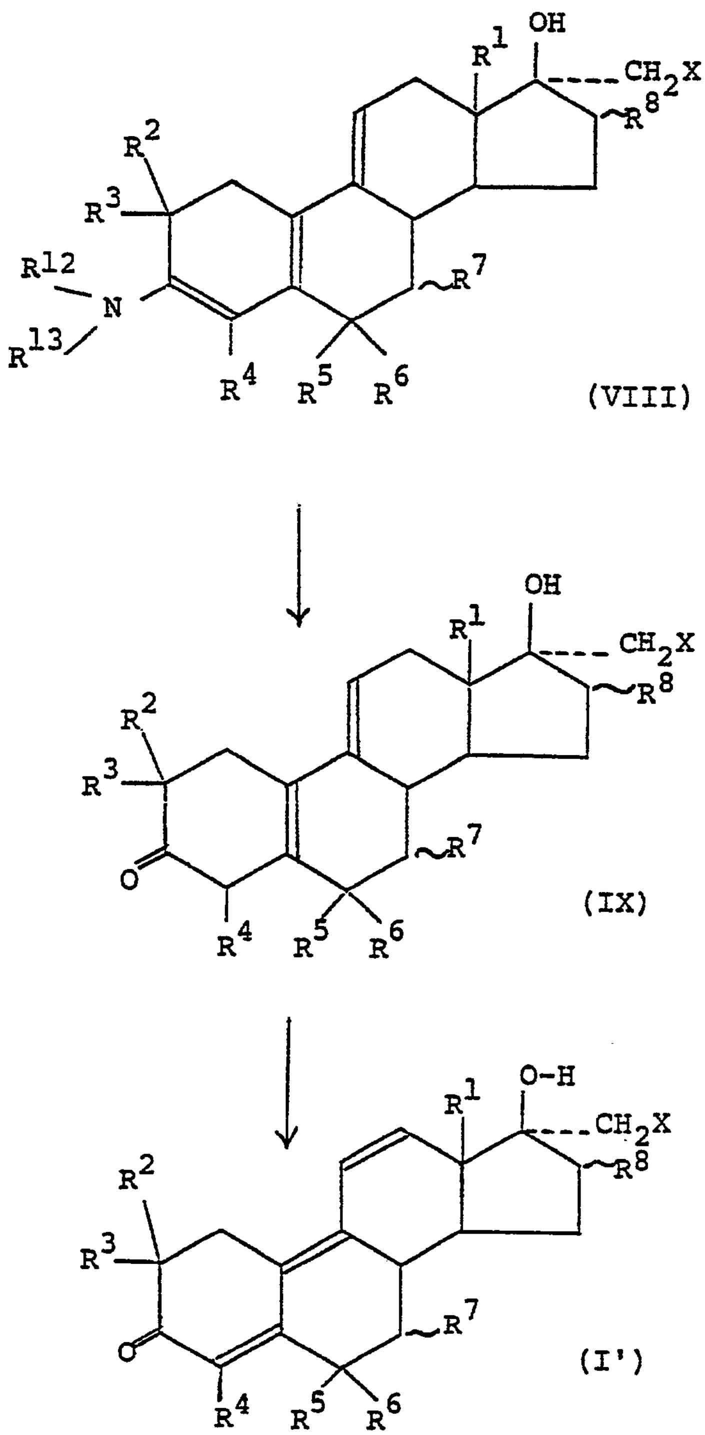

(VIII)

(IX)

(I')

The starting material in this second process is gonadiene having the following formula (VI) which can be prepared in the method described in Arzneimittelforschung/ Drug Res. 24 (1974) , pp. 896 - 900.

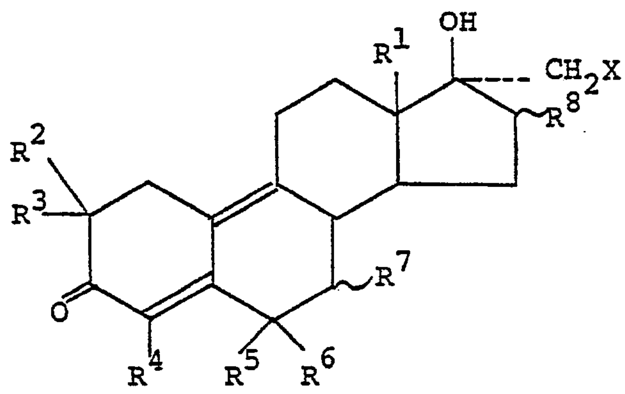

(VI)

wherein $R^1$ to $R^8$ and X are as herein defined.

Gonadiene (VI) is reacted with an amine having the formula (VII) :

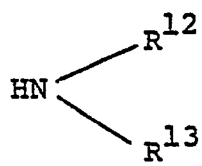

(VII)

wherein each of $R^{12}$ and $R^{13}$ is alkyl group having 1 to 4 carbon atoms or jointly form $-(CH_2)_n-$ where n is an integer of 2 to 4, such as dimethylamine, diethylamine or preferably pyrrolidine in a solvent, preferably lower alcohol such as methanol, ethanol or propanol to obtain enamine having the formula (VIII) :

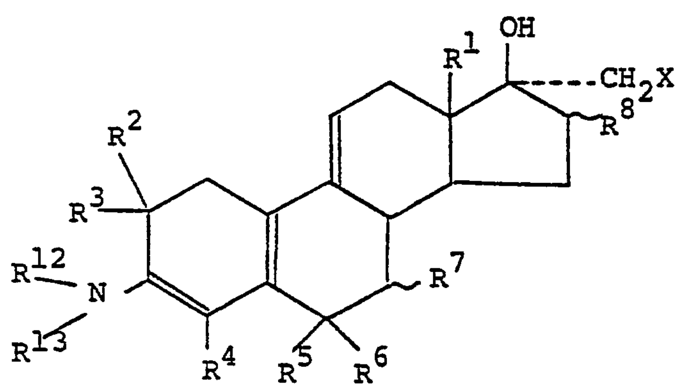

(VIII)

wherein $R^1$ to $R^8$, $R^{12}$, $R^{13}$ and X are as herein defined. The used amount of amine is at least 1 mole, preferably 1.5 to 3 moles per mole of gonadiene (VI). The reaction temperature is -10 to +70°C, preferably +10 to +40°C.

Then, enamine (VIII) is reacted with a lower fatty acid such as acetic acid, propionic acid or butyric acid to obtain diene having the formula (IX) :

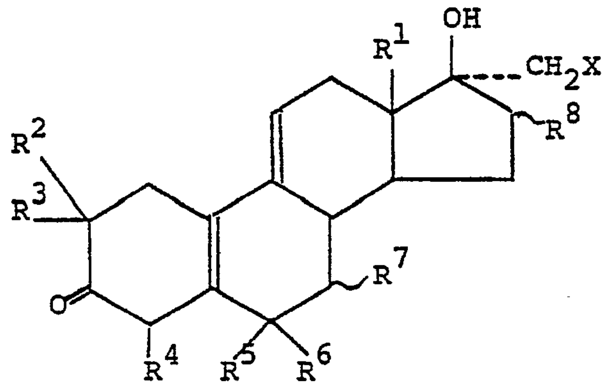

(IX)

wherein $R^1$ to $R^8$ and X are herein defined. The used amount of the lower fatty acid is a solvent amount. Preferably, water in a suitable amount, for example, 0.1 to 5 by volume per amount of the lower fatty acid, is co-exist in this reaction. The reaction temperature is -10 to +50°C, preferably -2 to +10°C.

Then, diene (IX) is dehydrogenated with dicyanodichlorobenzoquinone (DDQ) in a solvent, preferably an aprotic solvent such as cyclic ethers, for example, dioxane or tetrahydrofurane, to obtain gonatriene derivative (I′). The used amount of DDQ is at least 1 mole, preferably 1.5 to 3 moles per mole of diene (IX). The reaction temperature is 10 to 50°C, generally room temperature.

The gonatriene derivative of the general formula (I) wherein $R^1$ to $R^8$ and Y is as herein defined and X is $-SOR^9$ can be prepared by oxidizing the gonatriene derivative of the formula (I″) :

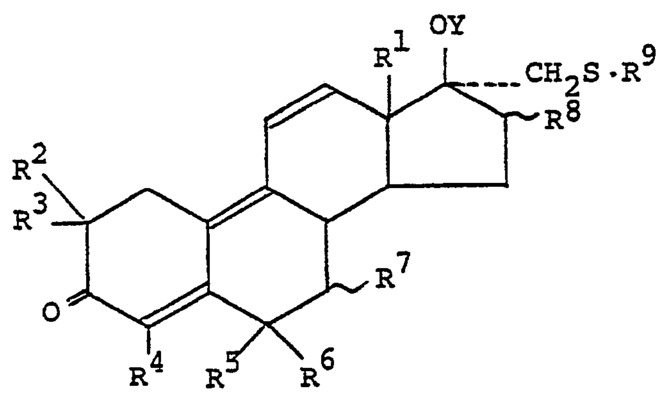

(I")

wherein $R^1$ to $R^8$ and Y are as herein defined, and prepared according to one of the above-mentioned two processes by oxidization using persalt, preferably sodium or potassium periodate in a solvent, preferably a mixed solvent of water and alcohol such as methanol or ethanol. The reaction temperature is preferably 10 to 30°C.

And, the gonatriene derivative of the general formula (I) wherein $R^1$ to $R^8$ and X are as herein defined and Y is $-COR^{10}$ or $-R^{11}$ can be prepared by subjecting the gonatriene derivative (I′) to acylation or etherification in any of the known methods.

Examples

The following examples illustrate the invention, but are not to be constructed as limiting the scope thereof.

Example 1:

Preparation of 17α-chloromethyl-17β-hydroxy-13β-methyl-gona-4,9,11-triene-3-one

Step A:

8.83 g (32.90 mmol) of 13β-methyl-gona-4,9,11-triene-3,17-dione was dissolved in 420 ml of tetrahydrofuran. Then, 22 ml of ethyleneglycol and a catalytic amount (800 mg) of p-toluene-sulfonic acid were added thereto and the whole was cooled to 0°C in an ice bath. After the addition of 43.8 ml of ethyl orthoformate and the stirring at 0°C in a nitrogen stream for 3 hours, 5 ml of triethylamine was added so as to stop the reaction. The reaction liquid was diluted with 300 ml of ethyl acetate and washed with an aqueous

saturated sodium hydrogen carbonate solution and an aqueous saturated saline solution, successively. The washed aqueous layer was extracted with 200 ml of methylene chloride and the resultant organic layer was dried over an anhydrous magnesium sulfate and distilled to dryness under reduced pressure to obtain 14.37 g of a crude product as a yellow oily substance, which was subjected to a silica gel column chromatography using a n-hexane-ethyl acetate mixed solvent as an eluent to obtain 7.09 g of 3-ethylenedioxy-13β-methyl-gona-4,9,11-triene-17-one as pale yellow crystals. Yield was 69.0 %.

Step B:

7.09 g (22.69 mmol) of 3-ethylenedioxy-13β-methyl-gona-4,9,11-triene-17-one obtained in the above Step A was dissolved in 70 ml of dimethylformamide. Then, 9.26 g of trimethylsulfonium iodide was added thereto and stirred at room temperature in a nitrogen stream for 5 minutes. After 6.37 g of potassium t-butoxide was added, the stirring at room temperature in a nitrogen stream was further continued for 45 minutes. The reaction liquid was diluted with 500 ml of ethyl acetate and washed with an aqueous saturated ammonium chloride solution and an aqueous saturated saline solution, successively. The washed aqueous layer was extracted with 300 ml of ethyl acetate and the resultant organic layer was dried over an anhydrous magnesium sulfate and distilled to dryness under reduced pressure to obtain 9.86 g of a crude product as a brown oily substance, which was subjected to a silica gel column chromatography using a n-hexane-ethyl acetate mixed solvent as an eluent to obtain 6.04 g of 3-ethylenedioxy-13β-methyl-gona-4,9,11-triene-17β-spiro-1′,2′-oxirane as an amorphous solid. Yield was 81.1 %.

Step C:

3.01 g (9.22 mmol) of 3-ethylenedioxy-13β-methyl-gona-4,9,11-triene-17β-spiro-1′,2′-oxirane obtained in the above Step B was dissolved in 120 ml of dimethylformamide. Then, a mixed solvent of 3.0 ml of concentrated hydrochloric acid and 25 ml of dimethylformamide was added dropwise at room temperature for 5 minutes. After the stirring at room temperature in a nitrogen stream for 45 minutes, the whole was cooled in an ice bath and 40 ml of an aqueous saturatated sodium hydrogen carbonate solution was added thereto so as to stop the reaction. The reaction liquid was diluted with 500 ml of ethyl acetate and washed with water and an aqueous saturated saline solution, successively. The washed aqueous layer was extracted with 400 ml of ethyl acetate and the resultant organic layer was dried over an anhydrous magnesium sulfate and distilled to dryness under reduced pressure to obtain 7.73 g of a crude product as a yellow oily substance, which was subjected to a silica gel column chromatography using a n-hexane-ethyl acetate mixed solvent as an eluent followed by the recyrstallization from a benzene-cyclohexane (1:1) mixed solvent to obtain 1.91 g of 17α-chloromethyl-17β-hydroxy-13β-methyl-gona-4,9,11-triene-3-one having the following chemical characteristics as pale yellow crystals. Yield was 65.0 %.

Melting point : 173.1 - 173.4°C

NMR ($CDCl_3$, 90 MHz)
δ : 1.03 (3H, S)
3.48 (1H, d, J=12 Hz)
3.70 (3H, d, J=12 Hz)
5.93 (1H, br-s)
6.25 (1H, d, J=9 Hz)
6.43 (1H, d, J=9 Hz)

IR (KBr) : 3450, 1640 $cm^{-1}$

Example 2:

Preparation of 17α-cyanomethyl-17β-hydroxy-13β-methyl-gona-4,9,11-triene-3-one

Step A:

9.45 g (30.34 mmol) of 17α-cyanomethyl-17β-hydroxy-13β-methyl-gona-4,9,-diene-3-one was dissolved in 250 ml of methanol and then 3.0 ml of pyrrolidine was added thereto followed by stirring at room temperature in a nitrogen stream for 3.5 hours. The reaction liquid having insoluble deposits was distilled to dryness under reduced pressure while maintaining the temperature of the water bath at not more than 40°C to obtain 12.53 g of 17α-cyanomethyl-17β-hydroxy-13β-methyl-3-(N-pyrrolidyl)-gona-3,5(10),9(11)-triene as a yellowish brown solid, which crude product was used in the next step without any purification.

Step B:

Into 12.53 g of the crude 17α-cyanomethyl-17β-hydroxy-13β-methyl-3-(N-pyrrolidyl)-gona-3,5(10), 9(11)-triene obtained in the above Step A, a mixture of 17.5 ml of glacial acetic acid and 7.5 ml of water was slowly added at 0°C. After the stirring at 0°C in a nitrogen stream for 15 minutes, 150 ml of water was added and then the whole was allowed to stand at 0°C for 30 minutes. Further, 150 ml of water was added

thereto and then the whole was allowed to stand at 0°C for 30 minutes. The reaction liquid was extracted with 900 ml of ethyl acetate. The resultant organic layer was washed with 1N-sodium hydroxide solution, 0.5 N-hydrochloric acid solution, an aqueous saturated sodium hydrogen carbonate solution and an aqueous saturated sodium hydrogen carbonate solution and an aqueous saturated saline solution successively, dried over an anhydrous magnesium sulfate and distilled to dryness under reduced pressure to obtain 9.82 g of 17α-cyanomethyl-17β-hydroxy-13β-methyl-gona-5(10),9(11)-diene-3-one as a brown oily substance, which crude product was used in the next step without any purification.

Step C:

9.82 g of the crude 17α-cyanomethyl-17β-hydroxy-13β-methyl-gona-5(10),9(11)-diene-3-one obtained in the above Step B was dissolved in 250 ml of dioxane, to which a solution of 28.63 g of 2,3-dichloro-5,6-dicyano-p-benzoquinone in 500 ml of dioxane was added dropwise at room temperature for 30 minutes. Then, the whole was stirred at room temperature in a nitrogen stream for 12 hours. The reaction liquid was passed through a column of 750 g of active alumina with 1500 ml of ethyl acetate so as to remove 2,3-dichloro-5,6-dicyano-p-hydroquinone and the excess 2,3-dichloro-5,6-dicyano-p-benzoquinone. The resultant filtrate was distilled to dryness under reduced pressure to obtain 2.15 g of a crude product as a brown solid, which was subjected to a silica gel column chromatography using an n-hexane-ethyl acetate mixed solvent as an eluent followed by the recrystallization from a n-hexane-ethyl acetate (1:2) mixed solvent to obtain 0.67 g of 17α-cyanomethyl-17β-hydroxy-13β-methyl-gona-4,9,11-triene-3-one having the following chemical characteristics as pale yellow crystals. Yield from Step A was 6.9 %.

Melting point : 153.9 - 154.4°C
NMR($CDCl_3$, 90 MHz)
δ : 1.03 (3H, S)
5.78 (1H, br-s)
6.28 (1H, d, I=9.6 Hz)
6.52 (1H, d, J=9.6 Hz)
IR (KBr) : 3450, 2230, 1640 $cm^{-1}$

Example 3:

Preparation of 17α-cyanomethyl-17β-hydroxy-13β-ethyl-gona-4,9,11-triene-3-one

Step A:

6.00 g (21.25 mmol) of 13β-ethyl-gona-4,9,11-triene-3,17-dione was dissolved in 315 ml of tetrahydrofuran. Then, 14.2 ml of ethyleneglycol and a catalytic amount (500 mg) of p-toluene sulfonic acid were added thereto and the whole was cooled to 0°C in an ice bath. After the addition of 28.3 ml of ethyl orthoformate and the stirring at 0°C in a nitrogen stream for 3 hours, 5 ml of triethylamine was added so as to stop the reaction. The reaction liquid was diluted with 300 ml of ethyl acetate and washed with an aqueous saturated sodium hydrogen carbonate solution and an aqueous saturated saline solution, successively. The washed aqueous layer was extracted with 400 ml of methylene chloride and the resultant organic layer was dried over an anhydrous magnesium sulfate and distilled to dryness under reduced pressure to obtain 9.58 g of a crude product as a yellow oily substance, which was subjected to a silica gel column chromatography using a n-hexane-ethyl acetate mixed solvent as an eluent to obtain 6.29 g of 3-ethylenedioxy-13β-ethyl-gona-4,9,11-triene-17-one as pale yellow crystals. Yield was 90.6 %.

Step B:

6.29 g (19.27 mmol) of 3-ethylenedioxy-13β-ethyl-gona-4,9,11-triene-17-one obtained in the above Step A was dissolved in 60 ml of dimethylformamide. Then, 7.86 g of trimethylsulfonium iodide was added thereto and stirred at room temperature in a nitrogen stream for 5 minutes. After 5.41 g of potassium t-butoxide was added, the stirring at room temperature in a nitrogen stream was further continued for 45 minutes. The reaction liquid was diluted with 500 ml of ethyl acetate and washed with an aqueous saturated ammonium chloride solution and an aqueous saturated saline solution, successively. The washed aqueous layer was extracted with 300 ml of ethyl acetate and the resultant organic layer was dried over an anhydrous magnesium sulfate and distilled to dryness under reduced pressure to obtain 8.17 g of a crude product as a brown oily substance, which was subjected to a silica gel column chromatography using a n-hexane-ethyl acetate mixed solvent as an eluent to obtain 5.63 g of 3-ethylenedioxy-13β-ethyl-gona-4,9,11-triene-17β-spiro-1′,2′-oxirane as an amorphous solid. Yield was 85.8 %.

Step C:

5.63 g (16.54 mmol) of 13β-ethyl-3-ethylenedioxy-gona-4,9,11-triene-17β-spiro-1′, 2′-oxirane obtained in the above Step B was dissolved in 130 ml of ethanol. Then, 4.05 g of sodium cyanide was added thereto

and the reaction mixture was stirred at room temperature for 5 days in a closed system. The reaction liquid was diluted with 400 ml of methylene chloride and washed with water and an aqueous saturated saline solution, successively. The washed aqueous layer was extracted with 400 ml of ethylene chloride and the resultant organic layer was dried over an anhydrous magnesium sulfate and distilled to dryness under reduced pressure to obtain 6.43 g of a crude product as a yellow solid, which was subjected to a silica gel column chromatography using an n-hexane-ethyl acetate mixed solvent as an eluent to obtain 3.79 g of 17α-cyanomethyl-13β-ethyl-3-ethylenedioxy-17β-hydroxy-gona-4,9,11-triene as pale yellow crystals. Yield was 62.3 %.

Step D:

3.00 g (8.16 mmol) of 17α-cyanomethyl-13β-ethyl-3-ethylenedioxy-17β-hydroxy-gona-4,9,11-triene obtained in the above Step C was dissolved in 150 ml of acetone, to which 30 ml of water and a catalytic amount (100 mg) of p-toluene sulfonic acid were added. After the stirring at room temperature for 10 minutes, 20 ml of an aqueous sodium hydrogen carbonate solution was added so as to stop the reaction. The reaction liquid was diluted with 400 ml of ethyl acetate and washed with water and an aqueous saturated saline solution, successively. The washed aqueous layer was extracted with 400 ml of ethylene chloride and the resultant organic layer was dried over an anhydrous mangnesium sulfate followed by distilling to dryness under reduced pressure to obtain 2.84 g of a crude product as a yellow oily substance, which was subjected to a silica gel column chromatography using an n-hexane-ethyl acetate mixed solvent as an eluent and recrystallized from an n-hexane-ethyl acetate (1:2) mixed solvent to obtain 1.699 g of 17α-cyanomethyl-13β-ethyl-17β-hydroxy-gona-4,9,11-triene-3-one having the following chemical characteristics as pale yellow crystals. Yield was 64.4 %.

Melting point : 192 - 195°C
NMR ($CDCl_3$, 90 MHz)
δ : 1.05 (3H, t, J=7.5 Hz)
5.81 (1H, br-s)
6.35 (1H, d, J=10.5 Hz)
6.66 (1H, d, J=10.5 Hz)
IR (KBr) : 3450, 2250, 1640 $cm^{-1}$

Example 4:

Preparation of 17α-chloromethyl-17β-hydroxy-13β-ethyl-gona-4,9,11-triene-3-one

Steps A and B:

The Steps A and B in Example 3 were repeated.

Step C:

481 mg (1.41 mmol) of 13β-ethyl-3-ethylenedioxy-gona-4,9,11-triene-17β-spiro-1',2-oxirane obtained in the above Step B was dissolved in 18 ml of dimethyl-formamide, to which a mixed solvent of 0.5 ml of concentrated hydrochloric acid and 3.8 ml of dimethylformamide was added dropwise at room temperature for 5 minutes. After the stirring at room temperature in a nitrogen stream for 20 minutes, the whole was cooled in an ice bath and 10 ml of an aqueous sodium hydrogen carbonate solution was added so as to stop the reaction. The reaction liquid was diluted with 100 ml of ethyl acetate and washed with water and an aqueous saturated saline solution, successively. The washed aqueous layer was extracted with 50 ml of ethyl acetate and the resultant organic layer was dried over an anhydrous magnesium sulfate followed by distilling to dryness under reduced pressure to obtain 675 mg of a crude product as a yellowish brown solid, which was subjected to a silica gel column chromatography using an n-hexane-ethyl acetate mixed solvent as an eluent to obtain 423 mg of 17α-chloromethyl-13β-ethyl-17β-hydroxy-gona-4,9,11-triene-3-one having the following chemical characteristics as pale yellow amorphous solid. Yield was 90.0 %.

NMR ($CDCl_3$, 60 MHz)
δ : 1.02 (3H, t, J=7 Hz)
3.49 (1H, d, J=11 Hz)
3.78 (1H, d, J=11 Hz)
5.75 (1H, br-s)
6.30 (1H, d, J=10 Hz)
6.57 (1H, d, J=10 Hz)
IR (KBr) : 3450, 1640 $cm^{-1}$

Example 5:

Preparation of 17α-methoxymethyl-17β-hydroxy-13β-methyl-gona-4,9,11-triene-3-one

Steps A and B:

The Steps A and B in Example 1 were repeated.

Step C:

500 mg (1.53 mmol) of 3-ethylenedioxy-13β-methyl-gona-4,9,11-triene-17β-spiro-1',2'-oxirane obtained in the above Step B was dissolved in 15 ml of methanol. Then, 1.14 g of sodium methoxide was added thereto and stirred at the refluxing temperature in a nitrogen stream for 2 hours. After the cooling to room temperature, the reaction liquid was diluted with 100 ml of ethyl acetate and washed with an aqueous saturated ammonium chloride solution and an aqueous saturated saline solution, successively. The washed aqueous layer was extracted with 50 ml of ethyl acetate and the resultant organic layers were combined followed by drying over an anhydrous magnesium sulfate and distilling to dryness under reduced pressure to obtain 0.57 g of 3-ethylenedioxy-17α-methoxymethyl-17β-hydroxy-13β-methyl-gona-4,9,11-triene as a yellowish brown oily substance, which crude product was used in the next step without any purification.

Step D:

0.57 g of the crude 3-ethylenedioxy-17α-methoxymethyl-17β-hydroxy-13β-methyl-gona-4,9,11-triene obtained in the above Step C was dissolved in 10 ml of acetone. Then, 1 ml of water and a catalytic amount (20 mg) of p-toluene sulfonic acid were added thereto. After the stirring at room temperature for 10 minutes, 3 ml of an aqueous sodium hydrogen carbonate solution was added so as to stop the reaction. The reaction liquid was diluted with 50 ml of ethyl acetate and washed with water and an aqueous saturated saline solution, successively. The washed aqueous layer was extracted with 25 ml of ethyl acetate. The resultant organic layers were combined followed by drying over an anhydrous magnesium sulfate and distilling to dryness under reduced pressure to obtain 0.48 g of a crude product as a yellowish brown oily substance, which was subjected to a silica gel column chromatography using an n-hexane-ethyl acetate mixed solvent as an eluent to 332 mg of 17α-methoxymethyl-17β-hydroxy-13β-methyl-gona-4,9,11-triene-3-one having the following chemical characteristics as an amorphous solid. Yield was 69 %.

NMR ($CDCl_3$, 90 MHz)
δ : 1.03 (3H, s)
3.18 (1H, d, J=9 Hz)
3.40 (3H, s)
3.45 (1H, d, J=9 Hz)
5.78 (1H, brs)
6.55 (2H, brs)
IR (KBr) : 3450, 1640 $cm^{-1}$

Example 6:

Preparation of 17α-methoxymethyl-17β-hydroxy-13β-ethyl-gona-4,9,11-triene-3-one

Steps A and B:

The Steps A and B in Example 3 were repeated.

Step C:

3.26 g (9.57 mmol) of 13β-ethyl 3-ethylenedioxy-gona-4,9,11-triene-17β-spiro-1',2'-oxirane obtained in the above Step B was dissolved in 100 ml of methanol. Then, 7.28 g of sodium methoxide was added thereto and stirred at the refluxing temperature in a nitrogen stream for 8 hours. After the cooling to room temperature, the reaction liquid was diluted with 400 ml of ethyl acetate and washed with an aqueous saturated ammonium chloride solution and an aqueous saturated saline solution, successively. The washed aqueous layer was extracted with 200 ml of ethyl acetate. The resultant organic layers were combined followed by drying over an anhydrous magnesium sulfate and distilling to dryness under reduced pressure to obtain 3.41 g of 13β-ethyl-3-ethylenedioxy-17α-methoxymethyl-17β-hydroxy-gona-4,9,11-triene as a yellowish brown oily substance, which crude product was used in the next step without any purification.

Step D:

3.41 g of the crude 13β-ethyl-3-ethylenedioxy-17α-methoxymethyl-17β-hydroxy-gona-4,9,11-triene obtained in the above Step C was dissolved in 150 ml of acetone. Then, 30 ml of water and a catalytic amount (100 mg) of p-toluene sulfonic acid were added thereto. After the stirring at room temperature for 10 minutes, 10 ml of an aqueous sodium hydrogen carbonate solution was added so as to stop the reaction. The reaction liquid was diluted with 400 ml of ethyl acetate and washed with water and an aqueous saturated saline solution, successively. The washed aqueous layer was extracted with 200 ml of ethyl acetate. The resultant organic layers were combined followed by drying over an anhydrous magnesium sulfate and distilling to dryness under reduced pressure to obtain 3.00 g of a crude product as a yellowish brown oily substance, which was subjected to a silica gel column chromatography using an n-hexane-ethyl acetate mixed solvent as an eluent to obtain 2.30 g of 17α-methoxymethyl-17β-hydroxy-13β-ethyl-gona-4,9,11-triene-3-one having the following chemical characteristics as an amorphous solid. Yield was 76 %.

NMR ($CDCl_3$, 90 MHz)
δ : 1.03 (3H, t, J=7.5 Hz)
3.18 (1H, d, J=9 Hz)
3.40 (3H, s)
3.45 (1H, d, J=9 Hz)
5.78 (1H, brs)
6.55 (2H, brs)
IR (KBr) : 3450, 1640 $cm^{-1}$

Example 7:

Preparation of 17α-methylthiomethyl-17β-hydroxy-13β-ethyl-gona-4,9,11-triene-3-one

Steps A and B:

The Steps A and B in Example 3 were repeated.

Step C:

3.58 g (10.3 mmol) of 13β-ethyl-3-ethylenedioxy-gona-4,9,11-triene-17β-spiro-1′,2′-oxirane obtained in the above Step B was dissolved in 150 ml of ethyleneglycol. Then, 12 ml of an aqueous 15 % sodium methyl mercaptan solution was added thereto and stirred at 60°C in a nitrogen stream for 3 hours. After the cooling to room temperature, the reaction liquid was diluted with 500 ml of ethyl acetate and washed with water and an aqueous saturated saline solution, successively. The washed aqueous layer was extracted with 200 ml of ethyl acetate. The resultant organic layers were combined followed by drying over an anhydrous magnesium sulfate and distilling to dryness under reduced pressure to obtain 9.70 g of 13β-ethyl-3-ethylenedioxy-17α-methylthiomethyl-17β-hydroxy-gona-4,9,11-triene as a brown oily substance, which crude product was used in the next step without any purification.

Step D:

9.70 g of the crude 13β-ethyl-3-ethylenedioxy-17α-methylthiomethyl-17β-hydroxy-gona-4,9,11-triene obtained in the above Step C was dissolved in 150 ml of acetone. Then, 30 ml of water and a catalytic amount (100 mg) of p-toluene sulfonic acid were added thereto. After the stirring at room temperature for 10 minutes, 10 ml of an aqueous sodium hydrogen carbonate solution was added so as to stop the reaction. The reaction liquid was diluted with 300 ml of ethyl acetate and washed with water and an aqueous saturated saline solution, successively. The washed aqueous layer was extracted with 150 ml of ethyl acetate. The resultant organic layers were combined followed by drying over an anhydrous magnesium sulfate and distilling to dryness under reduced pressure to obtain 5.44 g of a crude product as a brown oily substance, which was subjected to a silica gel column chromatography using an n-hexane-ethyl acetate mixed solvent as an eluent to obtain 2.29 g of 17α-methylthiomethyl-17β-hydroxy-13β-ethyl-gona-4,9,11-triene-3-one having the following chemical characteristics as an amorphous solid. Yield was 42 %.

NMR ($CDCl_3$, 90 MHz)
δ : 1.03 (3H, t, J=7.5 Hz)
2.20 (3H, s)
5.78 (1H, brs)
6.40 (1H, d, J=10.5 Hz)
6.62 (1H, d, J=10.5 Hz)
IR (KBr) : 3450, 1640 $cm^{-1}$

Example 8:

Preparation of 17α-methylsulfinylmethyl-17β-hydroxy-13β-ethyl-gona-4,9,11-triene-3-one

Steps A and B:

The Steps A and B in Example 3 were repeated.

Step C:

3.58 g (10.3 mmol) of 13β-ethyl-3-ethylenedioxy-gona-4,9,11-triene-17β-spiro-1',2'-oxirane obtained in the above Step B was dissolved in 150 ml of ethyleneglycol. Then, 12 ml of an aqueous 15 % sodium methyl mercaptan solution was added thereto and stirred at 60°C in a nitrogen stream for 3 hours. After the cooling to room temperature, the reaction liquid was diluted with 500 ml of ethyl acetate and washed with water and an aqueous saturated saline solution, successively. The washed aqueous layer was extracted with 200 ml of ethyl acetate. The resultant organic layers were combined followed by drying over an anhydrous magnesium sulfate and distilling to dryness under reduced pressure to obtain 9.70 g of 13β-ethyl-3-ethylenedioxy-17α-methylthiomethyl-17β-hydroxy-gona-4,9,11-triene as a brown oily substance, which crude product was used in the next step without any purification.

Step D:

9.70 g of the crude 13β-ethyl- 3-ethylenedioxy-17α-methylthiomethyl-17β-hydroxy-gona-4,9,11-triene obtained in the above Step C was dissolved in 150 ml of acetone. Then, 30 ml of water and a catalytic amount (100 mg) of p-toluene sulfonic acid were added thereto. After the stirring at room temperature for 10 minutes, 10 ml of an aqueous sodium hydrogen carbonate solution was added so as to stop the reaction. The reaction liquid was diluted with 300 ml of ethyl acetate and washed with water and an aqueous saturated saline solution, successively. The washed aqueous layer was extracted with 150 ml of ethyl acetate. The resultant organic layers were combined followed by drying over an anhydrous magnesium sulfate and distilling to dryness under reduced pressure to obtain 5.44 g of a crude product as a brown oily substance, which was subjected to a silica gel column chromatography using an n-hexane-ethyl acetate mixed solvent as an eluent to obtain 2.29 g of 17α-methylthiomethyl-17β-hydroxy-13β-ethyl-gona-4,9,11-triene-3-one as an amorphous solid. Yield was 42 %.

Step E:

1.61 g (4.67 mmol) of 13β-ethyl-17α-methylthiomethyl-17β-hydroxy-gona-4,9,11-triene-3-one obtained in the above Step D was dissolved in 300 ml of methanol. Then, a solution of 1.51 g (6.54 mmol) of periodate in 300 ml of water was added thereto and stirred at room temperature in a nitrogen stream for 2 days. The reaction liquid was extracted with 300 ml of chloroform and washed with water and an aqueous saturated saline solution, successively. The washed aqueous layer was extracted with 200 ml of chloroform. The resultant organic layers were combined followed by drying over an anhydrous magnesium sulfate and distilling to dryness under reduced pressure to obtain 1.10 g of a crude product as a yellow oily substance, which was subjected to a silica gel column chromatography using a chloroform-methanol mixed solvent as an eluent followed by the recrystallization from an n-hexane-ethyl acetate mixed solvent to obtain 0.85 g of 17α-methylsulfinylmethyl-17β-hydroxy-13β-ethyl-gona-4,9,11-triene-3-one having the following chemical characteristics as white crystals. Yield was 47 %.

Melting point : 194 - 197°C
NMR ($CDCl_3$, 90 MHz)
δ : 1.05 (3H, t, J=7.5 Hz)
2.65 (3H, s)
2.96 (1H, d, J=13.5 Hz)
3.15 (1H, d, J=13.5 Hz)
5.80 (1H, brs)
6.50 (1H, d, J=9 Hz)
6.68 (1H, d, J=9 Hz)
IR (KBr) : 3450, 1650 $cm^{-1}$

Example 9:

Preparation of 17α-thiocyanatemethyl-17β-hydroxy-13βethyl-gona-4,9,11-triene-3-one

Steps A and B:

The Steps A and B in Example 3 were repeated.

Step C:

1.82 g (5.35 mmol) of 13β-ethyl-3-ethylenedioxy-gona-4,9,11-triene-17β-spiro-1',2'-oxirane obtained in the above Step B was dissolved in 200 ml of methanol. Then, a solution of 8.67 g (107 mmol) of sodium thiocyanate in 50 ml of water and 2.5 ml of glacial acetic acid were added thereto and stirred at 55°C in a nitrogen stream for 3 days. After the cooling to room temperature, the reaction liquid was diluted with 300 ml of chloroform and washed with water and an aqueous saturated saline solution, successively. The washed aqueous layer was extracted with 100 ml of chloroform. The resultant organic layers were combined followed by drying over an anhydrous magnesium sulfate and distilling to dryness under reduced pressure to obtain a crude product as a yellowish brown amorphous solid, which was subjected to a silica gel column chromatography using an n-hexane-ethyl acetate mixed solvent as an eluent followed by the recrystallization from an n-hexane-ethyl acetate mixed solvent to obtain 0.76 g of 17α-thiocyanatemethyl-17β-hydroxy-13β-ethyl-gona-4,9,11-triene-3-one as white crystals. Yield was 39 %.

Melting point : 192 - 194°C

NMR ($CDCl_3$, 90 MHz)

δ : 1.05 (3H, t, J=7.5 Hz)

3.07 (1H, d, J=12 Hz)

3.57 (1H, d, J=12 Hz)

5.83 (1H, brs)

6.39 (1H, d, J=10.5 Hz)

6.69 (1H, d, J=10.5 Hz)

IR (KBr) : 3450, 2280, 1640 $cm^{-1}$

Example 10:

Preparation of 17α-cyanomethyl-17β-hydroxy-13β-ethyl-gona-4,9,11-triene-3-one 17β-acetate

0.70 g (2.2 mmol) of 17α-cyanomethyl-17β-hydroxy-13β-ethyl-gona-4,9,11-triene-3-one obtained in Example 3 was dissolved in 13 ml of pyridine. Then, 13 ml of anhydrous acetic acid and 130 mg of 4-dimethylamino pyridine were added thereto and stirred at 45°C in a nitrogen stream for 4 hours. After the cooling to room temperature, the reaction liquid was diluted with 50 ml of ethyl acetate and washed with 3N-hydrochloric acid solution, an aqueous saturated sodium hydrogen carbonate solution and an aqueous saturated saline solution, successively. The washed aqueous layer was extracted with 50 ml of ethyl acetate. The resultant organic layers were combined followed by drying over an anhydrous magnesium sulfate and distilling to dryness under reduced pressure to obtain 0.91 g of a crude product as a yellowish brown amorphous solid, which was subjected to a silica gel column chromatography using an n-hexane-ethyl acetate mixed solvent to obtain 0.55 g of 17α-cyanomethyl-17β-hydroxy-13β-ethyl-gona-4,9,11-triene-3-one as pale yellow amorphous crystals. Yield was 78 %.

Test Example:

Each compound obtained in Examples and suspended in a 10 % ethanol-containing cotton seed oil was orally administrated to female rats in a dose of 0.1, 1 or 10 mg/kg for 14 days. While, for the control group, only the cotton seed oil was orally administrated for the same period.

After the completion of the administration, the ovary of each rat was weighted. From the comparison of the weight in the test group with that in the control group, the anti-gonadotropic activity of the gonatriene compound of this invention was evaluated.

The results are shown in the following Table.

| Dose / Compound | 0.1 mg/kg | 1 mg/kg | 10 mg/kg |
|---|---|---|---|
| Ex. 1 | - | ± | ++ |
| 2 | - | + | ++ |
| 3 | - | + | ++ |
| 4 | - | + | ++ |
| 6 | ± | ± | ++ |
| 7 | - | ± | ++ |
| 9 | | ++ | ++ |

NOTE

- no effective
+ effective
++ very effective

Effect of the Invention

As clear from the results of Test Example, the gonatriene derivatives of this invention have high antigonadotropic activities and their toxicity is low. Therefore, they are very effective as the pharmaceutical agent for treating endometriosis.

## Claims

1. A gonatriene derivative having the general formula (I):

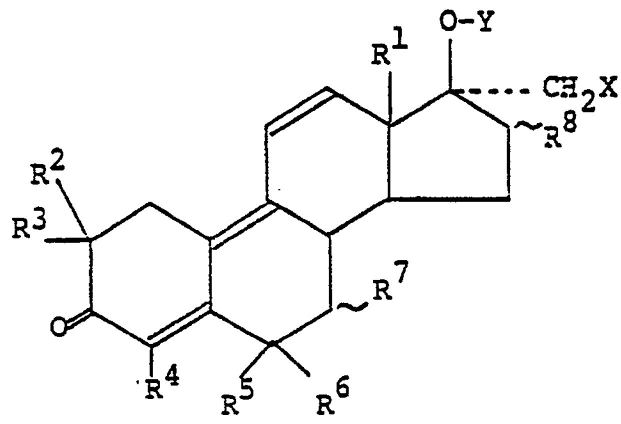

(I)

wherein
$R^1$ represents alkyl group having 1 to 3 carbon atoms;
X represents halogen atom, cyano group, $-OR^9$, $-SR^9$, $-SOR^9$ or –SCN where $R^9$ is alkyl group having 1 to 3 carbon atoms;
$R^2$, $R^3$, $R^4$ and $R^7$ independently represent hydrogen atom, halogen atom or alkyl group having 1 to 3 carbon atoms;
$R^5$ and $R^6$ independently represent hydrogen atom, halogen atom, alkyl group having 1 to 3 carbon atoms or hydroxy group, or jointly form oxo group;
$R^8$ represents hydrogen atom or alkyl group having 1 to 3 carbon atoms; and
Y represents a hydrogen atom, $-COR^{10}$ or $R^{11}$ where each of $R^{10}$ and $R^{11}$ is an alkyl group, an aryl group, a haloalkyl group or a haloaryl group, with the proviso that the above-defined groups cannot simultaneously have the following meanings: $R_2$, $R_3$, $R_4$, $R_5$, $R_6$, $R_7$, $R_8$ and Y represents hydrogen atoms, and X represents $-OCH_3$.

2. The compound of claim 1, wherein $R^1$ is methyl or ethyl.

3. The compound of claim 1, wherein X is chlorine atom, cyano group, $-OCH_3$, $-SCH_3$, $-SOCH_3$ or –SCN.

4. The compound of claim 1, wherein $R^2$ and $R^3$ are independently hydrogen atom or methyl group.

5. The compound of claim 1, wherein $R^4$ is hydrogen atom, chlorine atom or methyl group.

6. The compound of claim 1, wherein $R^5$ and $R^6$ are independently hydrogen atom, chlorine atom, methyl group or hydroxy group, or jointly form oxo group.

7. The compound of claim 1, wherein $R^7$ is hydrogen atom or methyl group.

8. The compound of claim 1, wherein $R^8$ is hydrogen atom, methyl group or ethyl group.

9. The compound of claim 1, wherein Y is hydrogen atom or $-COCH_3$ group.

10. A process for preparing a gonatriene derivative having the general formula (I):

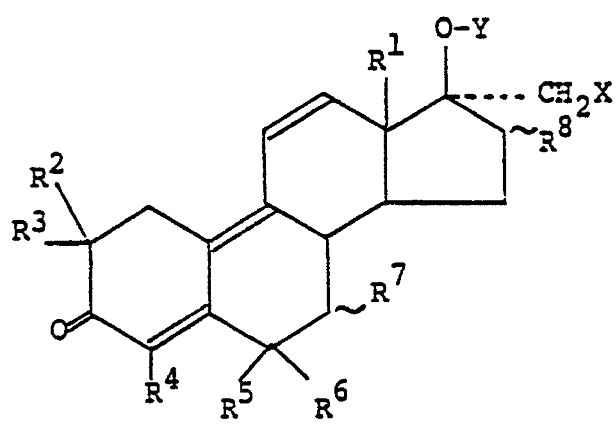

(I)

wherein
$R^1$ represents alkyl group having 1 to 3 carbon atoms;
X represents halogen atom, cyano group, $-OR^9$, $-SR^9$, or –SCN where $R^9$ is alkyl group having 1 to 3 carbon atoms;

$R^2$, $R^3$, $R^4$ and $R^7$ independently represent hydrogen atom, halogen atom or alkyl group having 1 to 3 carbon atoms;
$R^5$ and $R^6$ independently represent hydrogen atom, halogen atom, alkyl group having 1 to 3 carbon atoms or hydroxy group, or jointly form oxo group;
$R^8$ represents hydrogen atom or alkyl group having 1 to 3 carbon atoms; and
Y represents hydrogen atoms, $-COR^{10}$ or $R^{11}$ where each of $R^{10}$ and $R^{11}$ is alkyl group or aryl group optionally substituted with halogen atoms, with the proviso that the above-defined groups cannot simultaneously have the following meanings: $R_2$, $R_3$, $R_4$, $R_5$, $R_6$, $R_7$, $R_8$ and Y represents hydrogen atoms, and X represents $-OCH_3$;
which comprises the steps of:
reacting diketone of the formula (II):

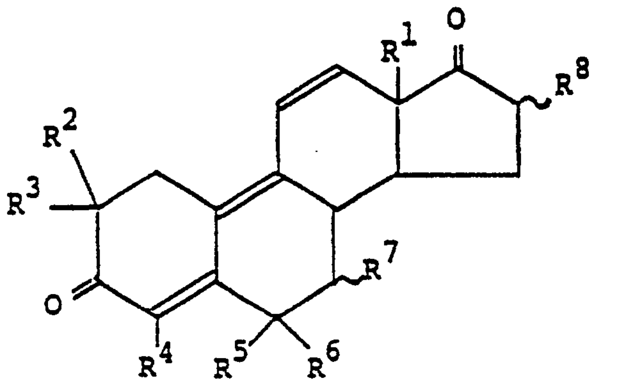

(II)

wherein $R^1$ to $R^8$ are as herein defined,
with ethylene glycol, trimethylene glycol or 2,2-dimethyltrimethylene glycol under an acidic condition to obtain acetal of the formula (III):

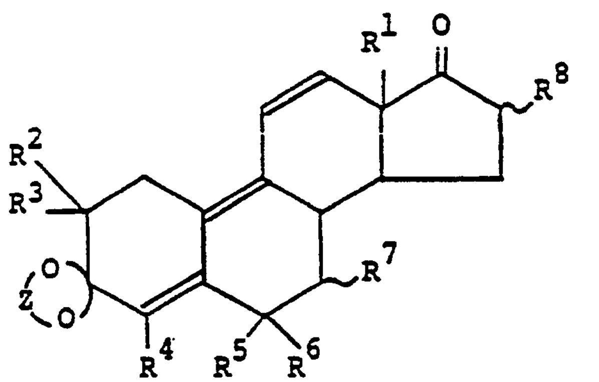

(III)

wherein $R^1$ to $R^8$ are as herein defined and Z is ethylene, trimethylene or 2,2-dimethyltrimethylene groups,
reacting the thus-obtained acetal (III) with dimethylsulfonium methylide or dimethyloxosulfonium methylide to obtain epoxy triene of the formula (IV):

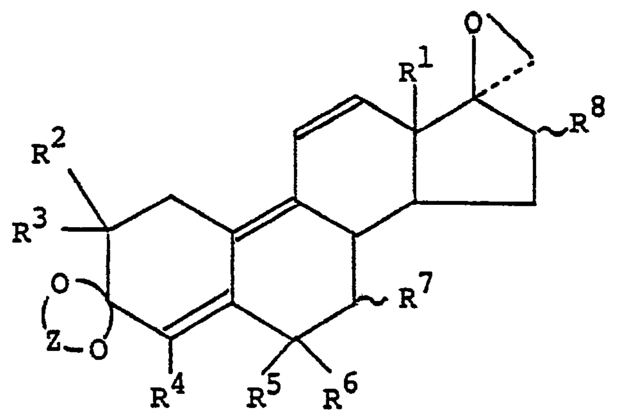

(IV)

wherein $R^1$ to $R^8$ and Z are as herein defined,
reacting the thus-obtained epoxy triene (IV) with cyanide, halide, metal alkoxide, metyl alkylsulfide or thiocyanate to obtain the compound of the formula (V):

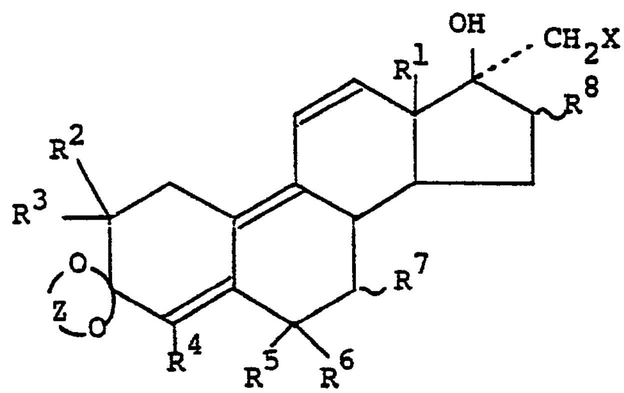

(V)

wherein $R^1$ to $R^8$, X and Z are as herein defined,
treating the thus-obtained compound (V) with an acid to obtain the gonatriene derivative of the formula (I'):

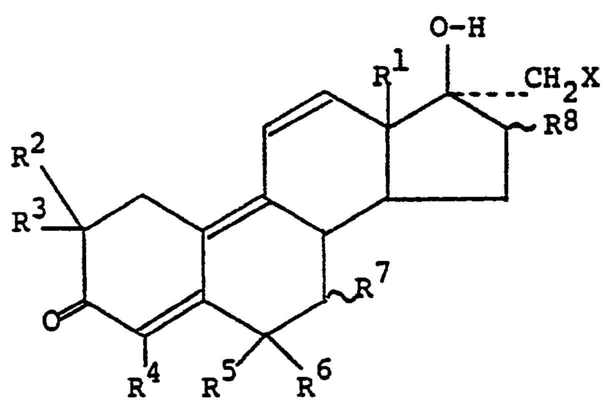

(I')

wherein $R^1$ to $R^8$ and X are as herein defined, and
if necessary, subjecting the gonatriene derivative (I') to acylation or etherification.

11. A process for preparing a gonatriene derivative having the general formula (I):

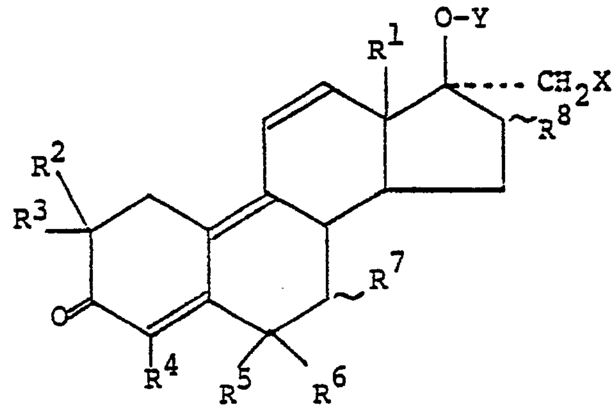

(I)

wherein

$R^1$ represents alkyl group having 1 to 3 carbon atoms;

X represents halogen atom, cyano group, or –SCN

$R^2$, $R^3$, $R^4$ and $R^7$ independently represent hydrogen atom, halogen atom or alkyl group having 1 to 3 carbon atoms;

$R^5$ and $R^6$ independently represent hydrogen atom, halogen atom, alkyl group having 1 to 3 carbon atoms or hydroxy group, or jointly form oxo group;

$R^8$ represents hydrogen atom or alkyl group having 1 to 3 carbon atoms; and

Y represents hydrogen atoms, $-COR^{10}$ or $R^{11}$ where each of $R^{10}$ and $R^{11}$ is alkyl group or aryl group optionally substituted with halogen atoms, with the proviso that the above-defined groups cannot simultaneously have the following meanings: $R_2$, $R_3$, $R_4$, $R_5$, $R_6$, $R_7$, $R_8$ and Y represents hydrogen atoms, and X represents $-OCH_3$;

which comprises the steps of:

reacting diketone of the formula (II):

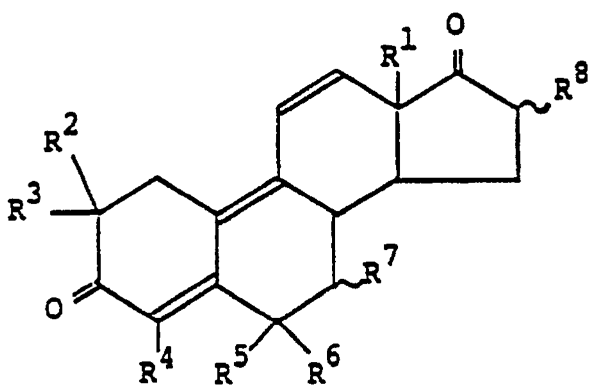

(II)

wherein $R^1$ to $R^8$ are as herein defined,

with ethylene glycol, trimethylene glycol or 2,2-dimethyltrimethylene glycol under an acidic condition to obtain acetal of the formula (III):

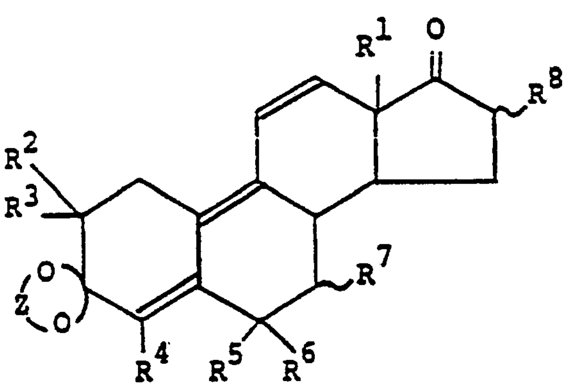

(III)

wherein $R^1$ to $R^8$ are as herein defined and Z is ethylene, trimethylene or 2,2-dimethyltrimethylene groups,
reacting the thus-obtained acetal (III) with dimethylsulfonium methylide or dimethyloxosulfonium methylide to obtain epoxy triene of the formula (IV):

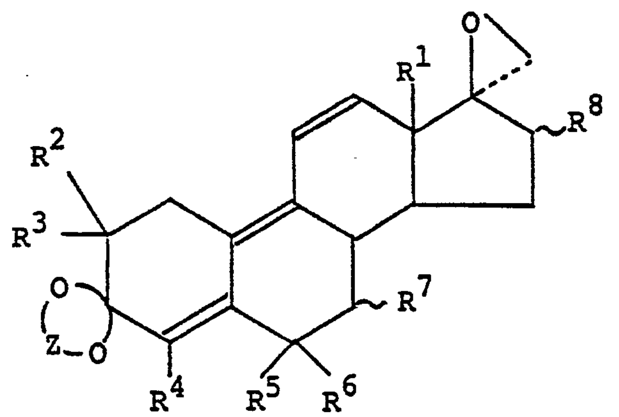

(IV)

wherein $R^1$ to $R^8$ and Z are as herein defined,
reacting the thus-obtained epoxy triene (IV) with cyanic acid, hydrogen halide or thiocyanic acid to obtain the gonatriene derivative of the formula (I'):

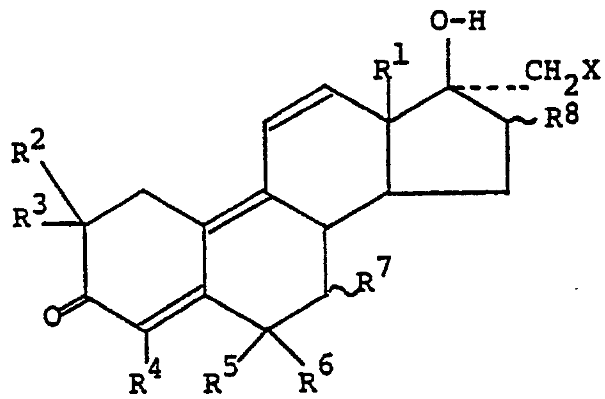

(I')

wherein $R^1$ to $R^8$ and X are as herein defined, and
if necessary, subjecting the thus-obtained gonatriene derivative (I') to acylation or etherification.

12. A process for preparing a gonatriene derivative having the general formula (I):

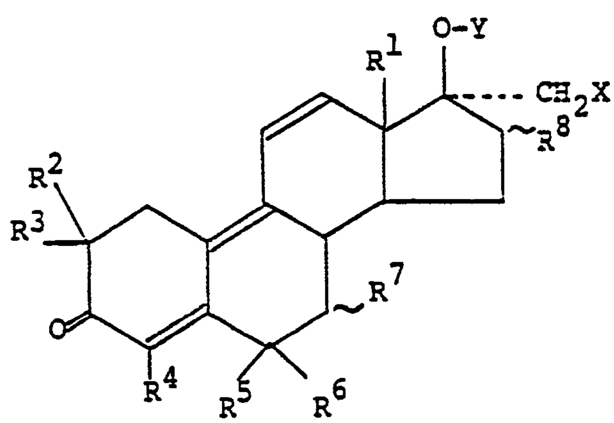

(I)

wherein
$R^1$ represents alkyl group having 1 to 3 carbon atoms;
X represents halogen atom, cyano group, $-OR^9$, $-SR^9$, $-SOR^9$ or $-SCN$ where $R^9$ is alkyl group having 1 to 3 carbon atoms;

$R^2$, $R^3$, $R^4$ and $R^7$ independently represent hydrogen atom, halogen atom or alkyl group having 1 to 3 carbon atoms;
$R^5$ and $R^6$ independently represents hydrogen atom, halogen atom, alkyl group having 1 to 3 carbon atoms or hydroxy group, or jointly form oxo group;
$R^8$ represents hydrogen atom or alkyl group having 1 to 3 carbon atoms; and
Y represents hydrogen atoms, $-COR^{10}$ or $R^{11}$ where each of $R^{10}$ and $R^{11}$ is alkyl group or aryl group optionally substituted with halogen atoms, with the proviso that the above-defined groups cannot simultaneously have the following meanings: $R_2$, $R_3$, $R_4$, $R_5$, $R_6$, $R_7$, $R_8$ and Y represents hydrogen atoms, and X represents $-OCH_3$;
which comprises the steps of:
reacting gonadiene of the formula (VI):

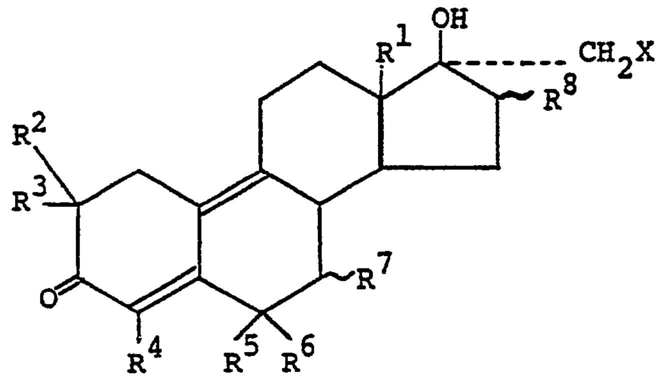

(VI)

wherein $R^1$ to $R^8$ and X are as herein defined,
with an amine of the formula (VII):

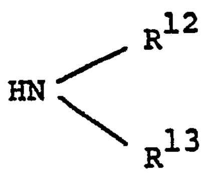

(VII)

wherein $R^{12}$ and $R^{13}$ independently represent alkyl group having 1 to 4 carbon atoms or jointly form $-(CH_2)_n-$ where n is an integer of 2 to 4,
to obtain enamine of the formula (VIII):

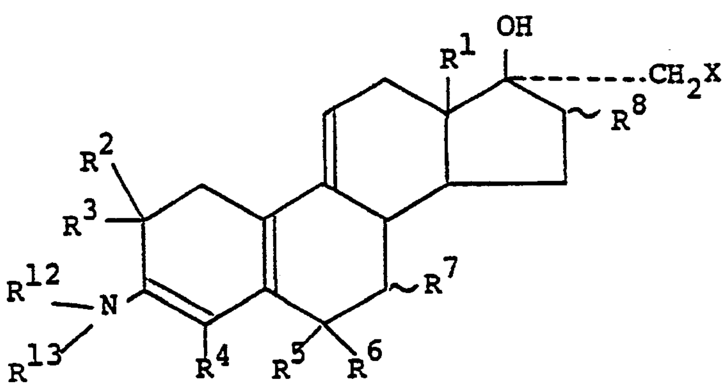

(VIII)

wherein $R^1$ to $R^8$, $R^{12}$, $R^{13}$ and X are as herein defined,
treating the thus-obtained enamine (VIII) with an acid to obtain diene of the formula (IX):

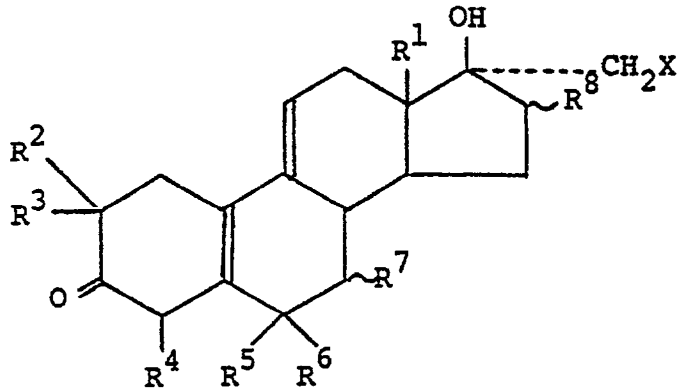

(IX)

wherein $R^1$ to $R^8$ and X are a herein defined,
reacting the thus-obtained diene (IX) with dichlorodicyanobenzoquinone to obtain the gonatriene derivative of the formula (I'):

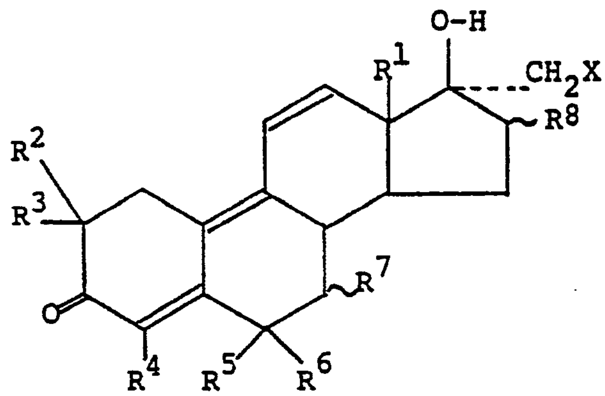

(I')

wherein $R^1$ to $R^8$ and X are as herein defined, and
if necessary, subjecting the thus-obtained gonatriene derivative (I') to acylation or etherification.

13. A process for preparing a gonatriene derivative having the general formula (I):

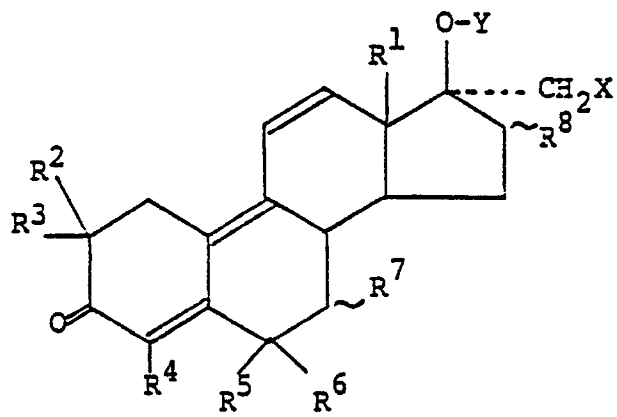

(I)

wherein
$R^1$ represents alkyl group having 1 to 3 carbon atoms;
X represents $-SOR^9$;
$R^2$, $R^3$, $R^4$ and $R^7$ independently represent hydrogen atom, halogen atom or alkyl group having 1 to 3 carbon atoms;
$R^5$ and $R^6$ independently represent hydrogen atom, halogen atom, alkyl group having 1 to 3 carbon atoms or hydroxy group, or jointly form oxo group;
$R^8$ represents hydrogen atom or alkyl group having 1 to 3 carbon atoms; and
Y represents hydrogen atoms, $-COR^{10}$ or $R^{11}$ where each of $R^{10}$ and $R^{11}$ is alkyl group or aryl group optionally substituted with halogen atoms, with the proviso that the above-defined groups cannot simultaneously have the following meanings: $R_2$, $R_3$, $R_4$, $R_5$, $R_6$, $R_7$, $R_8$ and Y represents hydrogen atoms, and X represents $-OCH_3$;
which comprises oxidizing the gonatriene derivative of the formula (I″):

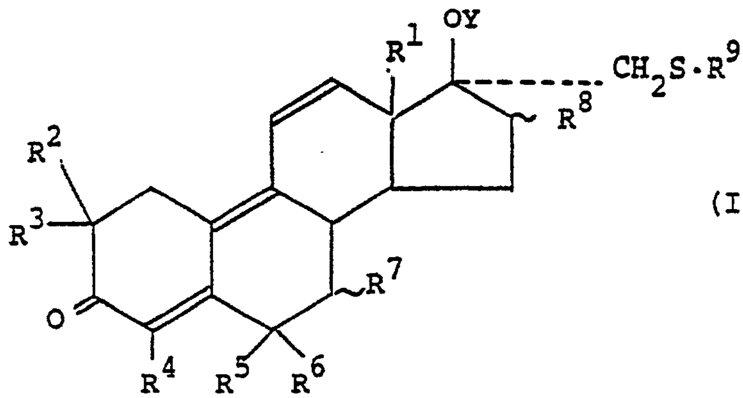

(I")

wherein $R^1$ to $R^9$ and Y are as herein defined.

14. A pharmaceutical agent for treating the endometriosis containing a gonatriene derivative of the general formula (I):

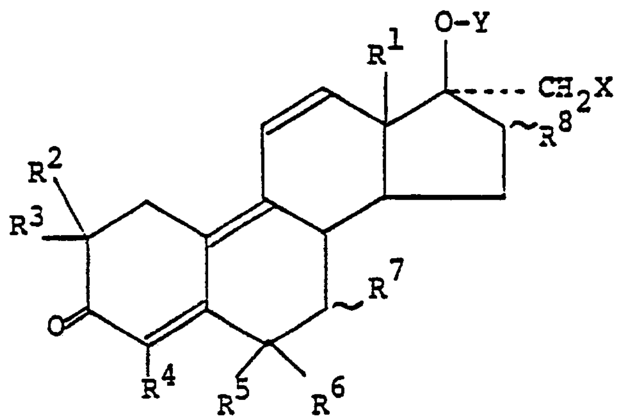

(I)

wherein
$R^1$ represents alkyl group having 1 to 3 carbon atoms;
X represents halogen atom, cyano group, $-OR^9$, $-SR^9$, $-SOR^9$ or $-SCN$ where $R^9$ is alkyl group having 1 to 3 carbon atoms;
$R^2$, $R^3$, $R^4$ and $R^7$ independently represent hydrogen atom, halogen atom or alkyl group having 1 to 3 carbon atoms;
$R^5$ and $R^6$ independently represent hydrogen atom, halogen atom, alkyl group having 1 to 3 carbon atoms or hydroxy group, or jointly form oxo group;
$R^8$ represents hydrogen atom or alkyl group having 1 to 3 carbon atoms; and
Y represents hydrogen atoms, $-COR^{10}$ or $R^{11}$ where each of $R^{10}$ and $R^{11}$ is an alkyl group, an aryl group, a haloalkyl group or a haloaryl group, with the proviso that the above-defined groups cannot simultaneously have the following meanings: $R_2$, $R_3$, $R_4$, $R_5$, $R_6$, $R_7$, $R_8$ and Y represents hydrogen atoms and X represents $-OCH_3$.

15. Use of a gonatriene derivative for the preparation of pharmaceutical compositions for the treatment of endometriosis having the general formula (I):

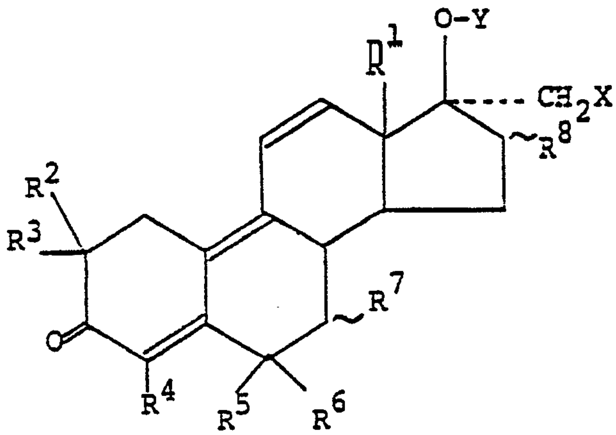

(I)

wherein
$R^1$ represents alkyl group having 1 to 3 carbon atoms;
X represents halogen atom, cyano group, $-OR^9$, $-SR^9$, $-SOR^9$ or $-SCN$ where $R^9$ is alkyl group having 1 to 3 carbon atoms;
$R^2$, $R^3$, $R^4$ and $R^7$ independently represent hydrogen atom, halogen atom or alkyl group having 1 to 3 carbon atoms;
$R^5$ and $R^6$ independently represent hydrogen atom, halogen atom, alkyl group having 1 to 3 carbon atoms or hydroxy group, or jointly form oxo group;
$R^8$ represents hydrogen atom or alkyl group having 1 to 3 carbon atoms; and
Y represents hydrogen atoms, $-COR^{10}$ or $R^{11}$ where each of $R^{10}$ and $R^{11}$ is an alkyl group, an aryl group, a haloalkyl group or a haloaryl group.

**Patentansprüche**

1. Gonatrien-Derivat der allgemeinen Formel (I)

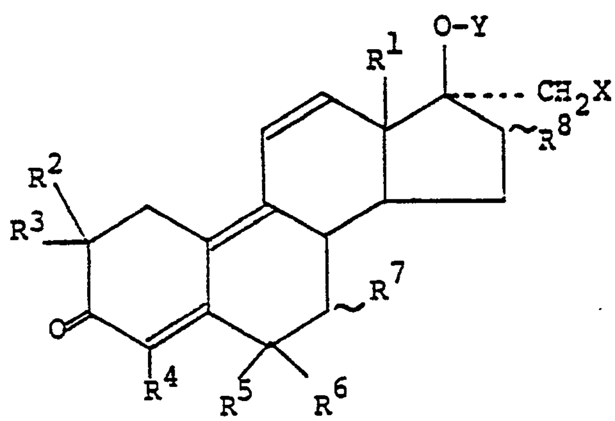

(I)

in der
$R^1$ eine Alkylgruppe mit 1 bis 3 Kohlenstoffatomen;
X ein Halogenatom, eine Cyanogruppe oder eine Gruppe der Formeln $-OR^9$, $-SR^9$, $-SOR^9$ oder $-SCN$, worin $R^9$ eine Alkylgruppe mit 1 bis 3 Kohlenstoffatomen darstellt;
$R^2$, $R^3$, $R^4$ und $R^7$ unabhängig voneinander Wasserstoffatome, Halogenatome oder Alkylgruppen mit 1 bis 3 Kohlenstoffatomen;
$R^5$ und $R^6$ unabhängig voneinander Wasserstoffatome, Halogenatome, Alkylgruppen mit 1 bis 3 Kohlenstoffatomen oder Hydroxylgruppen oder gemeinsam eine Oxogruppe;
$R^8$ ein Wasserstoffatom oder eine Alkylgruppe mit 1 bis 3 Kohlenstoffatomen; und
Y ein Wasserstoffatom, eine Gruppe der Formel $-COR^{10}$ oder $R^{11}$, worin $R^{10}$ und $R^{11}$ jeweils Alkylgruppen, Arylgruppen, Halogenalkylgruppen oder Halogenarylgruppen darstellen, mit der Maßgabe bedeuten, daß die oben definierten Gruppen nicht gleichzeitig die folgenden Bedeutungen besitzen:
$R^2$, $R^3$, $R^4$, $R^5$, $R^6$, $R^7$, $R^8$ und Y bedeuten Wasserstoffatome und X bedeutet eine Gruppe der Formel $-OCH_3$.

2. Verbindung nach Anspruch 1, worin $R^1$ eine Methylgruppe oder eine Ethylgruppe bedeutet.

3. Verbindung nach Anspruch 1, worin X ein Chloratom, eine Cyanogruppe oder eine Gruppe der Formeln $-OCH_3$, $-SCH_3$, $-SOCH_3$ oder $-SCN$ bedeutet.

4. Verbindung nch Anspruch 1, worin $R^2$ und $R^3$ unabhängig voneinander Wasserstoffatome oder Methylgruppen darstellen.

5. Verbindung nach Anspruch 1, worin $R^4$ ein Wasserstoffatom, ein Chloratom oder eine Methylgruppe bedeutet.

6. Verbindung nach Anspruch 1, worin $R^5$ und $R^6$ unabhängig voneinander Wasserstoffatome, Chloratome, Methylgruppen oder Hydroxylgruppen oder gemeinsam eine Oxogruppe bedeuten.

7. Verbindung nach Anspruch 1, worin $R^7$ ein Wasserstoffatom oder eine Methylgruppe darstellt.

8. Verbindung nach Anspruch 1, worin $R^8$ ein Wasserstoffatom, eine Methylgruppe oder eine Ethylgruppe bedeutet.

9. Verbindung nach Anspruch 1, worin Y ein Wasserstoffatom oder eine Gruppe der Formel $-COCH_3$ bedeutet.

10. Verfahren zur Herstellung eines Gonatrien-Derivates der allgemeinen Formel (I)

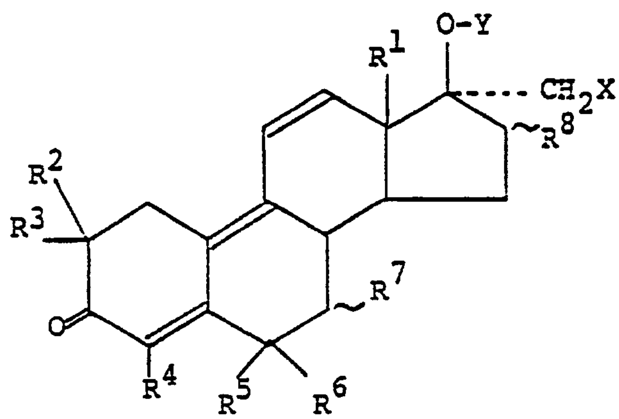

(I)

in der

$R^1$ eine Alkylgruppe mit 1 bis 3 Kohlenstoffatomen;

X ein Halogenatom, eine Cyanogruppe oder eine Gruppe der Formeln $-OR^9$, $-SR^9$ oder $-SCN$, worin $R^9$ eine Alkylgruppe mit 1 bis 3 Kohlenstoffatomen darstellt;

$R^2$, $R^3$, $R^4$ und $R^7$ unabhängig voneinander Wasserstoffatome, Halogenatome oder Alkylgruppen mit 1 bis 3 Kohlenstoffatomen;

$R^5$ und $R^6$ unabhängig voneinander Wasserstoffatome, Halogenatome, Alkylgruppen mit 1 bis 3 Kohlenstoffatomen oder Hydroxylgruppen oder gemeinsam eine Oxogruppe;

$R^8$ ein Wasserstoffatom oder eine Alkylgruppe mit 1 bis 3 Kohlenstoffatomen; und

Y ein Wasserstoffatom, eine Gruppe der Formel $-COR^{10}$ oder $R^{11}$, worin $R^{10}$ und $R^{11}$ jeweils gegebenenfalls halogensubstituierte Alkylgruppen oder Arylgruppen darstellen, mit der Maßgabe bedeuten, daß die oben definierten Gruppen nicht gleichzeitig die folgenden Bedeutungen besitzen können:

$R^2$, $R^3$, $R^4$, $R^5$, $R^6$, $R^7$, $R^8$ und Y bedeuten Wasserstoffatome und X bedeutet eine Gruppe der Formel $-OCH_3$;

welches folgende Schritte umfaßt:

Umsetzen eines Diketons der Formel (II)

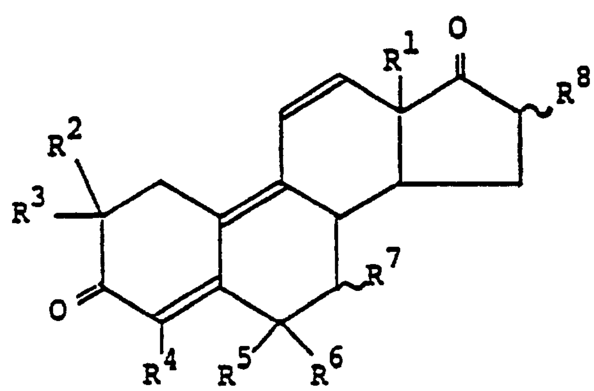

(II)

worin $R^1$ bis $R^8$ die oben angegebenen Bedeutungen besitzen, mit Ethylenglykol, Trimethylenglykol oder 2,2-Dimethyl-trimethylenglykol unter sauren Bedingungen zur Bildung eines Acetals der Formel (III)

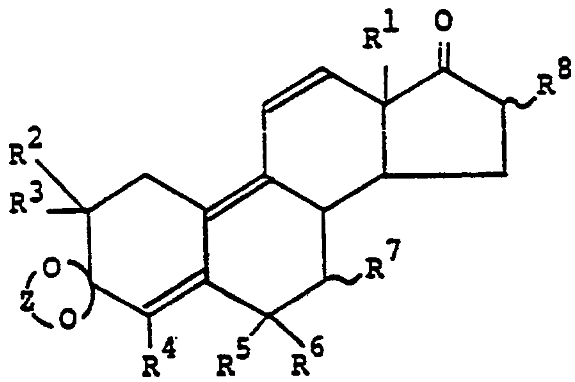

(III)

in der $R^1$ bis $R^8$ die oben angegebenen Bedeutungen besitzen und Z eine Ethylen-, Trimethylen- oder 2,2-Dimethyltrimethylengruppe darstellt,
Umsetzen des in dieser Weise erhaltenen Acetals (III) mit Dimethylsulfoniummethylid oder Dimethyloxosulfoniummethylid zur Bildung eines Epoxytriens der Formel (IV)

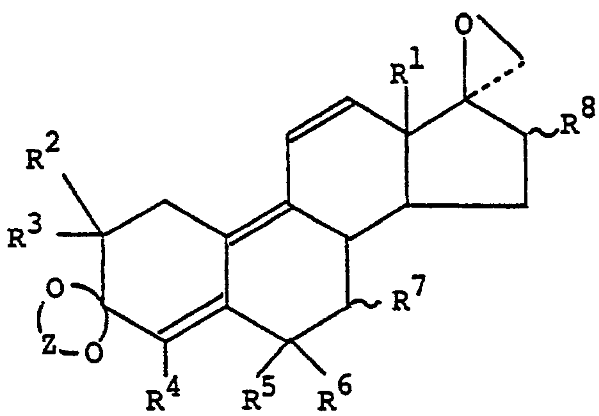

(IV)

in der $R^1$ bis $R^8$ und Z die oben angegebenen Bedeutungen besitzen,
Umsetzen des in dieser Weise erhaltenen Epoxytriens (IV) mit einem Cyanid, Halogenid, Metallalkoxid, Metallalkylsulfid oder Thiocyanat zur Bildung einer Verbindung der Formel (V)

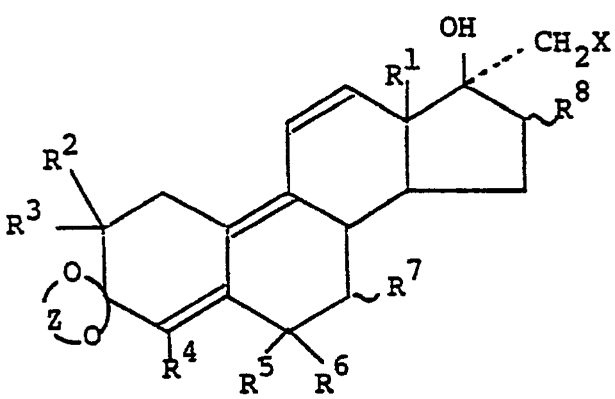

(V)

in der $R^1$ bis $R^8$, X und Z die oben angegebenen Bedeutungen besitzen,
Behandeln der in dieser Weise erhaltenen Verbindung (V) mit einer Säure zur Bildung des Gonatrien-Derivates der Formel (I'):

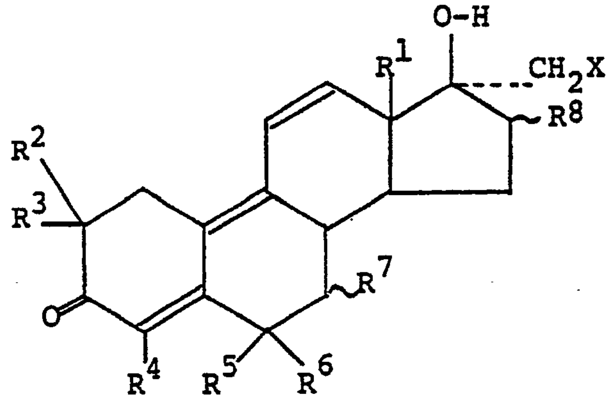

(I')

in der $R^1$ bis $R^8$ und X die oben angegebenen Bedeutungen besitzen, und erforderlichenfalls Acylieren oder Verethern des Gonatrien-Derivats (I').

11. Verfahren zur Herstellung eines Gonatrien-Derivats der allgemeinen Formel (I)

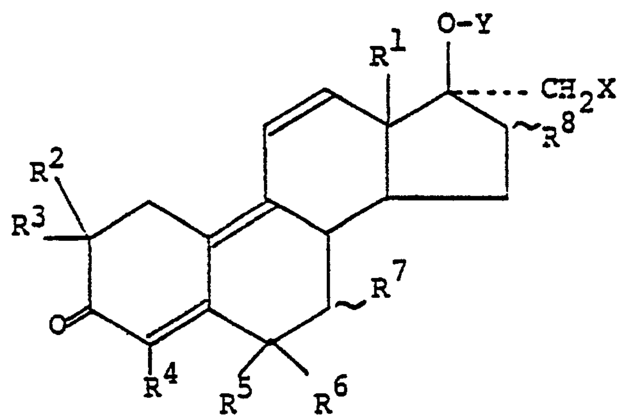

(I)

in der
$R^1$ eine Alkylgruppe mit 1 bis 3 Kohlenstoffatomen;
X ein Halogenatom, eine Cyanogruppe oder eine Gruppe der Formel –SCN;
$R^2$, $R^3$, $R^4$ und $R^7$ unabhängig voneinander Wasserstoffatome, Halogenatome oder Alkylgruppen mit 1 bis 3 Kohlenstoffatomen;
$R^5$ und $R^6$ unabhängig voneinander Wasserstoffatome, Halogenatome, Alkylgruppen mit 1 bis 3 Kohlenstoffatomen oder Hydroxylgruppen oder gemeinsam eine Oxogruppe;
$R^8$ ein Wasserstoffatom oder eine Alkylgruppe mit 1 bis 3 Kohlenstoffatomen; und
Y ein Wasserstoffatom oder eine Gruppe der Formel $-COR^{10}$ oder $R^{11}$, worin $R^{10}$ und $R^{11}$ jeweils gegebenenfalls halogensubstituierte Alkylgruppen oder Arylgruppen darstellen, mit der Maßgabe bedeuten, daß die oben definierten Gruppen nicht gleichzeitig die folgenden Bedeutungen besitzen können:
$R^2$, $R^3$, $R^4$, $R^5$, $R^6$, $R^7$, $R^8$ und Y bedeuten Wasserstoffatome und X bedeutet eine Gruppe der Formel $-OCH_3$;
welches folgende Schritte umfaßt:
Umsetzen eines Diketons der Formel (II)

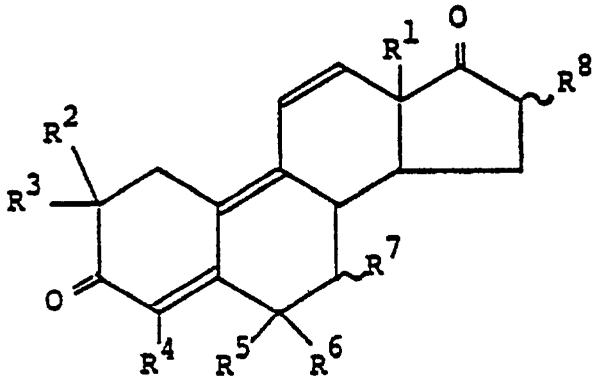

(II)

in der $R^1$ bis $R^8$ die oben angegebenen Bedeutungen besitzen, mit Ethylenglykol, Trimethylenglykol oder 2,2-Dimethyltrimethylenglykol unter sauren Bedingungen zur Bildung eines Acetals der Formel (III)

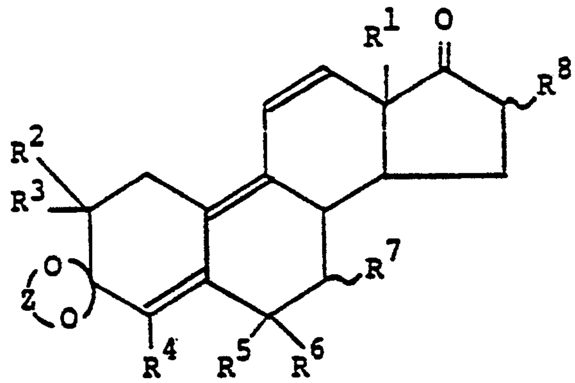

(III)

in der $R^1$ bis $R^8$ die oben angegebenen Bedeutungen besitzen und Z eine Ethylen-, Trimethylen- oder 2,2-Dimethyltrimethylengruppe bedeutet,
Umsetzen des in dieser Weise erhaltenen Acetals (III) mit Dimethylsulfoniummethylid oder Dimethyloxosulfoniummethylid zur Bildung eines Epoxytriens der Formel (IV)

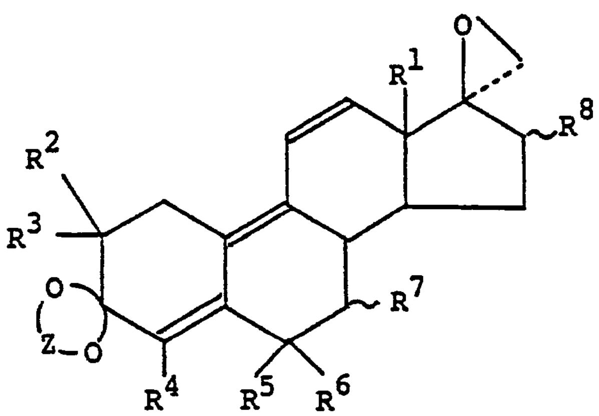

(IV)

in der $R^1$ bis $R^8$ und Z die oben angegebenen Bedeutungen besitzen,
Umsetzen des in dieser Weise erhaltenen Epoxytriens (IV) mit Cyansäure, Halogenwasserstoff oder Thiocyansäure zur Bildung der Gonatrien-Derivate der Formel (I'):

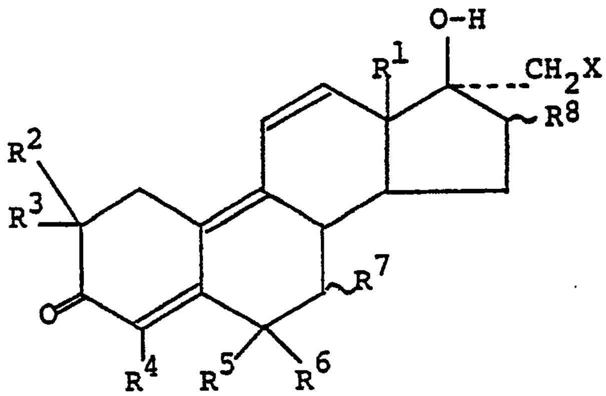

(I')

in der $R^1$ bis $R^8$ und X die oben angegebenen Bedeutungen besitzen und gegebenenfalls Acylieren oder Verethern der in dieser Weise erhaltenen Gonatrien-Detivate (I').

12. Verfahren zur Herstellung von Gonatrien-Derivaten der allgemeinen Formel (I)

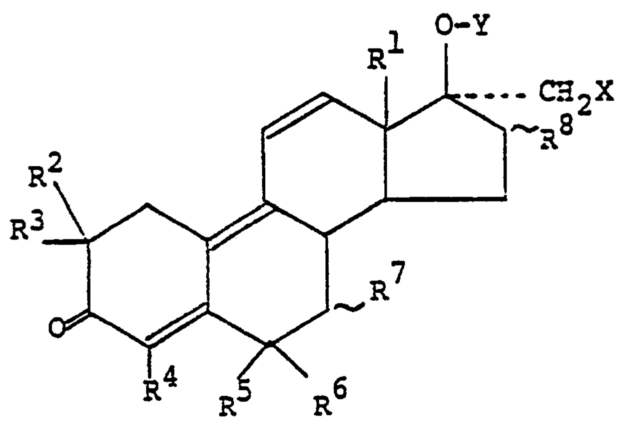

(I)

in der
$R^1$ eine Alkylgruppe mit 1 bis 3 Kohlenstoffatomen,
X ein Halogenatom, eine Cyanogruppe oder eine Gruppe der Formeln $-OR^9$, $-SR^9$, $-SOR^9$ oder $-SCN$, worin $R^9$ eine Alkylgruppe mit 1 bis 3 Kohlenstoffatomen darstellt;
$R^2$, $R^3$, $R^4$ und $R^7$ unabhängig voneinander Wasserstoffatome, Halogenatome oder Alkylgruppen mit 1 bis 3 Kohenstoffatomen;
$R^5$ und $R^6$ unabhängig voneinander Wasserstoffatome, Halogenatome, Alkylgruppen mit 1 bis 3 Kohlenstoffatomen oder Hydroxylgruppen oder gemeinsam eine Oxogruppe;
$R^8$ ein Wasserstoffatom oder eine Alkylgruppe mit 1 bis 3 Kohlenstoffatomen;
Y ein Wasserstoffatom oder eine Gruppe der Formel $-COR^{10}$ oder $R^{11}$, worin $R^{10}$ und $R^{11}$ jeweils gegebenenfalls halogensubstituierte Alkylgruppen oder Arylgruppen darstellen, mit der Maßgabe bedeuten, daß die oben definierten Gruppen nicht gleichzeitig die folgenden Bedeutungen besitzen können:
$R^2$, $R^3$, $R^4$, $R^5$, $R^6$, $R^7$, $R^8$ und Y bedeuten Wasserstoffatome und X bedeutet eine Gruppe der Formel $-OCH_3$,
welches die folgenden Schritte umfaßt:
Umsetzen eines Gonadiens der Formel (VI)

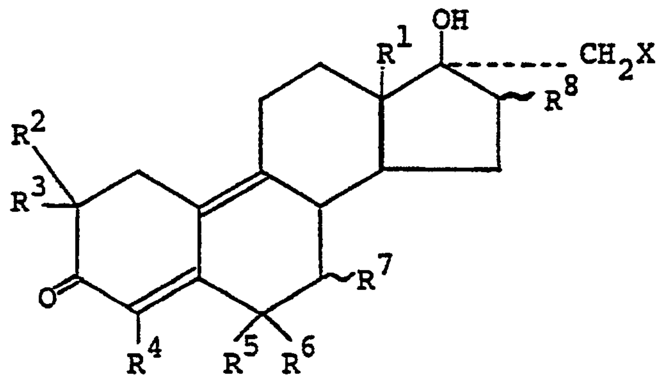

(VI)

worin $R^1$ bis $R^8$ und X die oben angegebenen Bedeutungen besitzen, mit einem Amin der Formel (VII)

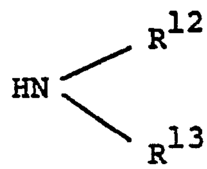

(VII)

worin $R^{12}$ und $R^{13}$ unabhängig voneinander Alkylgruppen mit 1 bis 4 Kohlenstoffatomen oder gemeinsam eine Gruppe der Formel $-(CH_2)_n-$ worin n für eine ganze Zahl mit einem Wert von 2 bis 4 steht, bedeuten. zur Bildung eines Enamins der Formel (VIII)

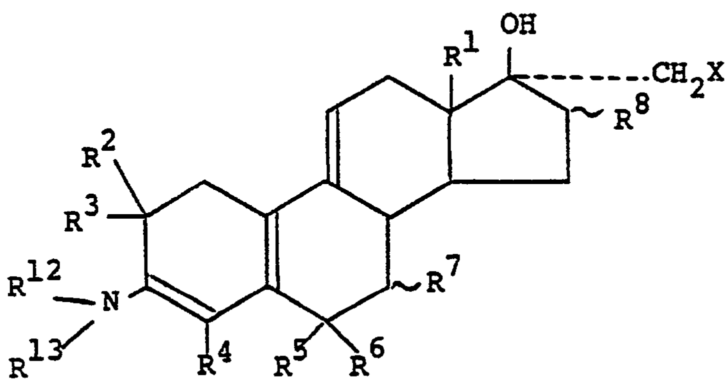

(VIII)

in der $R^1$ bis $R^8$, $R^{12}$, $R^{13}$ und X die oben angegebenen Bedeutungen besitzen,
Umsetzen des in dieser Weise erhaltenen Enamins (VIII) mit einer Säure zur Bildung eines Diens der Formel (IX)

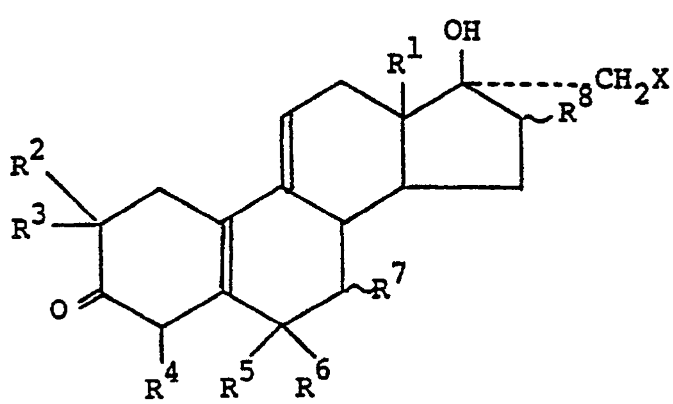

(IX)

in der $R^1$ bis $R^8$ und X die oben angegebenen Bedeutungen besitzen,

Umsetzen des in dieser Weise erhaltenen Diens (IX) mit Dichlordicyanobenzochinon zur Bildung des Gonatrien-Derivats der Formel (I'):

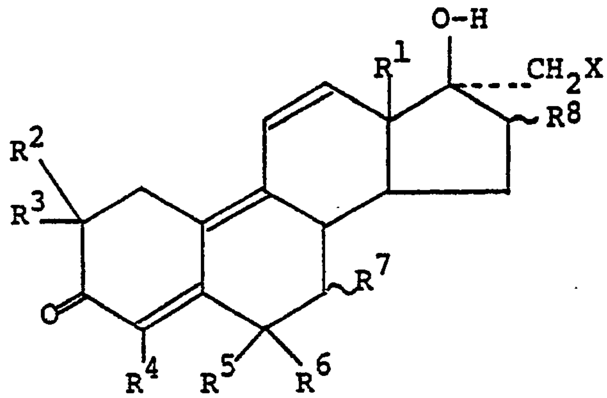

(I')

in der $R^1$ bis $R^8$ und X die oben angegebenen Bedeutungen besitzen, und
gegebenenfalls Acylieren oder Verethern des in dieser Weise erhaltenen Gonatrien-Derivats (I').

13. Verfahren zur Herstellung eines Gonatrien-Derivats der allgemeinen Formel (I)

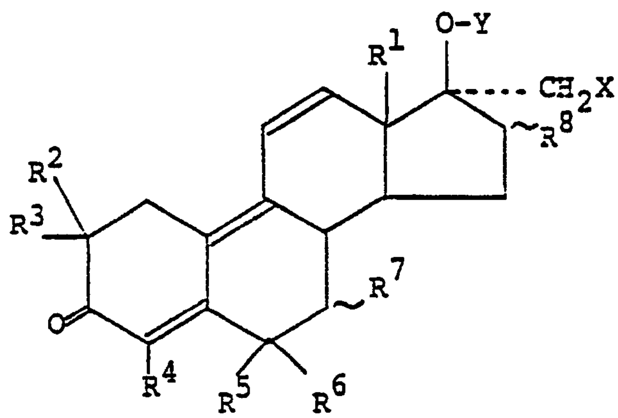

(I)

in der
$R^1$ eine Alkylgruppe mit 1 bis 3 Kohlenstoffatomen;
X eine Gruppe der Formel $-SOR^9$;
$R^2$, $R^3$, $R^4$ und $R^7$ unabhängig voneinander Wasserstoffatome, Halogenatome oder Alkylgruppen mit 1 bis 3 Kohlenstoffatomen;
$R^5$ und $R^6$ unabhängig voneinander Wasserstoffatome, Halogenatome, Alkylgruppen mit 1 bis 3 Kohlenstoffatomen oder Hydroxylgruppen oder gemeinsam eine Oxogruppe;
$R^8$ ein Wasserstoffatom oder eine Alkylgruppe mit 1 bis 3 Kohlenstoffatomen; und
Y ein Wasserstoffatom oder eine Gruppe der Formel $-COR^{10}$ oder $R^{11}$, worin $R^{10}$ und $R^{11}$ jeweils gegebenenfalls Halogen-substituierte Alkylgruppen oder Arylgruppen darstellen, mit der Maßgabe bedeuten, daß die oben definierten Gruppen nicht gleichzeitig die folgenden Bedeutungen besitzen können:
$R^2$, $R^3$, $R^4$, $R^5$, $R^6$, $R^7$, $R^8$ und Y bedeuten Wasserstoffatome und X bedeutet eine Gruppe der Formel $-OCH_3$;
welches darin besteht, ein Gonatrien-Derivat der Formel (I''):

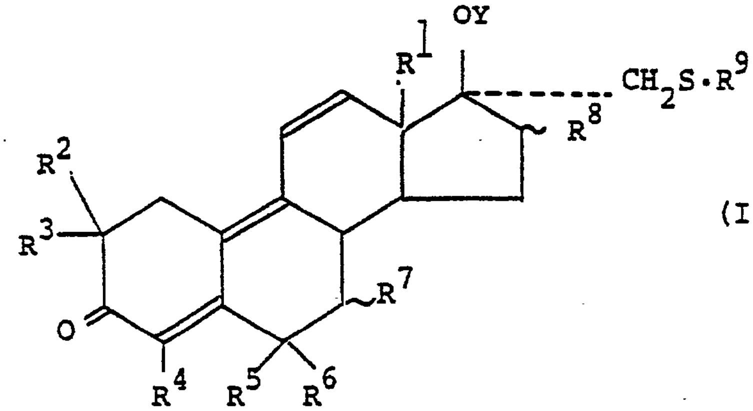

(I")

in der $R^1$ bis $R^9$ und Y die oben angegebenen Bedeutungen besitzen, zu oxidieren.

14. Pharmazeutisches Mittel zur Behandlung von Endometriose enthaltend ein Gonatrien-Derivat der allgemeinen Formel (I):

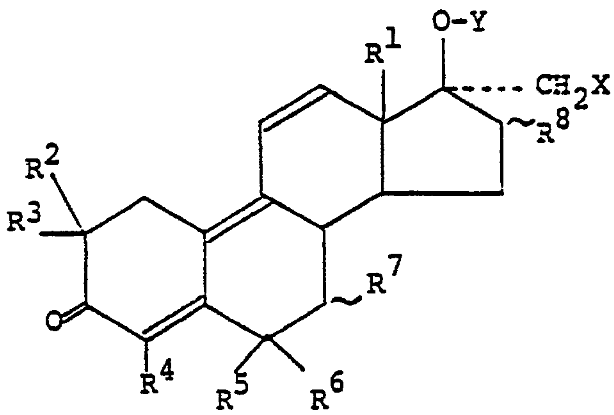

(I)

in der

$R^1$ eine Alkylgruppe mit 1 bis 3 Kohlenstoffatomen;

X ein Halogenatom, eine Cyanogruppe oder eine Gruppe der Formeln $-OR^9$, $-SR^9$, $-SOR^9$ oder $-SCN$, worin $R^9$ eine Alkylgruppe mit 1 bis 3 Kohlenstoffatomen darstellt;

$R^2$, $R^3$, $R^4$ und $R^7$ unabhängig voneinander Wasserstoffatome, Halogenatome oder Alkylgruppen mit 1 bis 3 Kohlenstoffatomen;

$R^5$ und $R^6$ unabhängig voneinander Wasserstoffatome, Halogenatome, Alkylgruppen mit 1 bis 3 Kohlenstoffatomen oder Hydroxylgruppen oder gemeinsam eine Oxogruppe;

$R^8$ ein Wasserstoffatom oder eine Alkylgruppe mit 1 bis 3 Kohlenstoffatomen;

Y ein Halogenatom, eine Gruppe der Formel $-COR^{10}$ oder $R^{11}$, worin $R^{10}$ und $R^{11}$ jeweils Alkylgruppen, Arylgruppen, Halogenalkylgruppen oder Halogenarylgruppen darstellen, mit der Maßgabe bedeuten, daß die oben definierten Gruppen nicht gleichzeitig die folgenden Bedeutungen besitzen:

$R^2$, $R^3$, $R^4$, $R^5$, $R^6$, $R^7$, $R^8$ und Y bedeuten Wasserstoffatome und X bedeutet eine Gruppe der Formel $-OCH_3$.

15. Verwendung eines Gonatrien-Derivats der allgemeinen Formel (I)

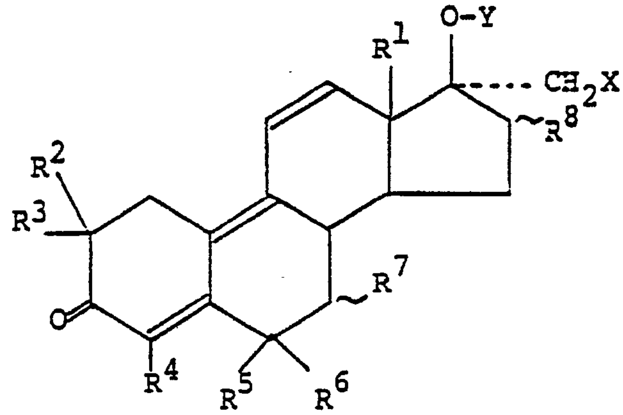

(I)

in der
$R^1$ eine Alkylgruppe mit 1 bis 3 Kohlenstoffatomen;
X ein Halogenatom, eine Cyanogruppe oder eine Gruppe der Formeln $-OR^9$, $-SR^9$, $-SOR^9$ oder $-SCN$, worin $R^9$ eine Alkylgruppe mit 1 bis 3 Kohlenstoffatomen darstellt;
$R^2$, $R^3$, $R^4$ und $R^7$ unabhängig voneinander Wasserstoffatome, Halogenatome oder Alkylgruppen mit 1 bis 3 Kohlenstoffatomen;
$R^5$ und $R^6$ unabhängig voneinander Wasserstoffatome, Halogenatome, Alkylgruppen mit 1 bis 3 Kohlenstoffatomen oder Hydroxylgruppen oder gemeinsam eine Oxogruppe;
$R^8$ ein Wasserstoffatom oder eine Alkylgruppe mit 1 bis 3 Kohlenstoffatomen;
Y ein Wasserstoffatom, eine Gruppe der Formel $-COR^{10}$ oder $R^{11}$, worin $R^{10}$ und $R^{11}$ jeweils Alkylgruppen, Arylgruppen, Halogenalkylgruppen oder Halogenarylgruppen darstellen, bedeuten,
zur Herstellung von pharmazeutischen Zubereitungen zur Behandlung der Endometriose.

**Revendications**

1. Dérivé de gonatriène représenté par la formule générale (I):

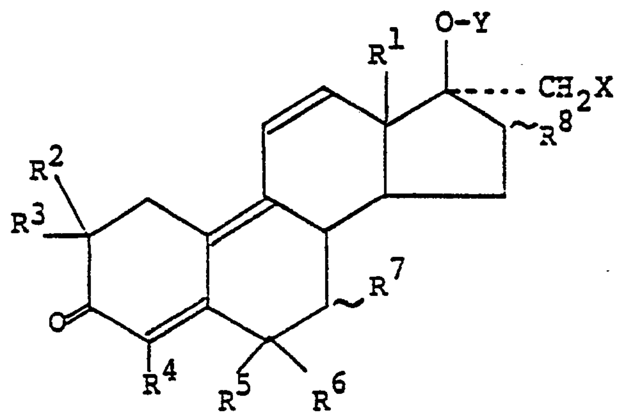

(I)

dans laquelle:
$R^1$ représente un groupe alkyle ayant 1 à 3 atomes de carbone;
X représente un atome d'halogène, un groupe cyano, $-OR^9$, $-SR^9$, $-SOR^9$ ou $-SCN$, où $R^9$ est un groupe alkyle ayant 1 à 3 atomes de carbone;
$R^2$, $R^3$, $R^4$ et $R^7$ représentent indépendamment un atome d'hydrogène, un atome d'halogène ou un groupe alkyle ayant 1 à 3 atomes de carbone;
$R^5$ et $R^6$ représentent indépendamment un atome d'hydrogène, un atome d'halogène, un groupe alkyle ayant 1 à 3 atomes de carbone ou un groupe hydroxy, ou bien forment conjointement un groupe oxo;
$R^8$ représente un atome d'hydrogène ou un groupe alkyle ayant 1 à 3 atomes de carbone; et
Y représente un atome d'hydrogène, $-COR^{10}$ ou $R^{11}$, où $R^{10}$ et $R^{11}$ représentent chacun un groupe alkyle, un groupe aryle, un groupe haloalkyle ou un groupe haloaryle,
à la condition que les groupes définis ci-dessus ne peuvent pas avoir simultanément les significations suivantes:
$R_2$, $R_3$, $R_4$, $R_5$, $R_6$, $R_7$, $R_8$ et Y représentent des atomes d'hydrogène; et
X représente $-OCH_3$.

2. Composé selon la revendication 1, dans lequel $R^1$ représente méthyle ou éthyle.

3. Composé selon la revendication 1, dans lequel X représente un atome de chlore, un groupe cyano, $-OCH_3$, $-SCH_3$, $-SOCH_3$ ou $-SCN$.

4- Composé selon la revendication 1, dans lequel $R^2$ et $R^3$ représentent indépendamment un atome d'hydrogène ou un groupe méthyle.

5. Composé selon la revendication 1, dans lequel $R^4$ est un atome d'hydrogène, un atome de chlore ou un groupe méthyle.

6. Composé selon la revendication 1, dans lequel $R^5$ et $R^6$ représentent indépendamment un atome d'hydrogène, un atome de chlore, un groupe méthyle ou un groupe hydroxy, ou bien forment conjointement un groupe oxo.

7. Composé selon la revendication 1, dans lequel $R^7$ représente un atome d'hydrogène ou un groupe méthyle.

8. Composé selon la revendication 1, dans lequel $R^8$ représente un atome d'hydrogène, un groupe méthyle ou un groupe éthyle.

9. Composé selon la revendication 1, dans lequel Y représente un atome d'hydrogène ou un groupe $-COCH_3$.

10. Procédé de préparation d'un dérivé de gonatriène représenté par la formule générale (I):

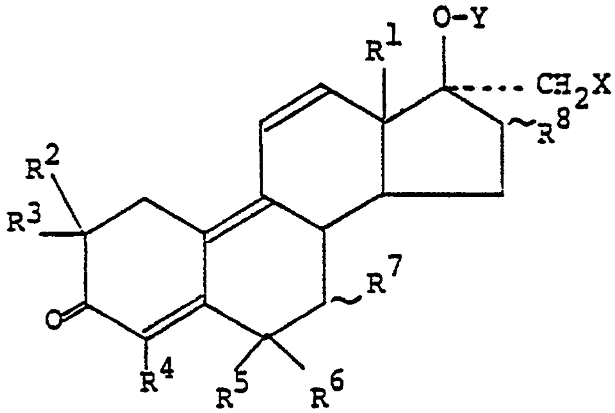

(I)

dans laquelle:
$R^1$ représente un groupe alkyle ayant 1 à 3 atomes de carbone;
X représente un atome d'halogène, un groupe cyano $-OR^9$, $-SR^9$, ou $-SCN$, où $R^9$ est un groupe alkyle ayant 1 à 3 atomes de carbone;
$R^2$, $R^3$, $R^4$ et $R^7$ représentent indépendamment un atome d'hydrogène, un atome d'halogène ou un groupe alkyle ayant 1 à 3 atomes de carbone;
$R^5$ et $R^6$ représentent indépendamment un atome d'hydrogène, un atome d'halogène, un groupe alkyle ayant 1 à 3 atomes de carbone ou un groupe hydroxy, ou bien forment conjointement un groupe oxo;
$R^8$ représente un atome d'hydrogène ou un groupe alkyle ayant 1 à 3 atomes de carbone; et
Y représente un atome d'hydrogène, $-COR^{10}$ ou $R^{11}$, où $R^{10}$ et $R^{11}$ représentent chacun un groupe alkyle ou un groupe aryle facultativement substitués par des atomes d'halogène,
à la condition que les groupes définis ci-dessus ne peuvent pas avoir simultanément les significations suivantes:
$R_2$, $R_3$, $R_4$, $R_5$, $R_6$, $R_7$, $R_8$ et Y représentent des atomes d'hydrogène; et
X représente $-OCH_3$,
qui comprend les étapes consistant à:
faire réagir une dicétone de formule (II):

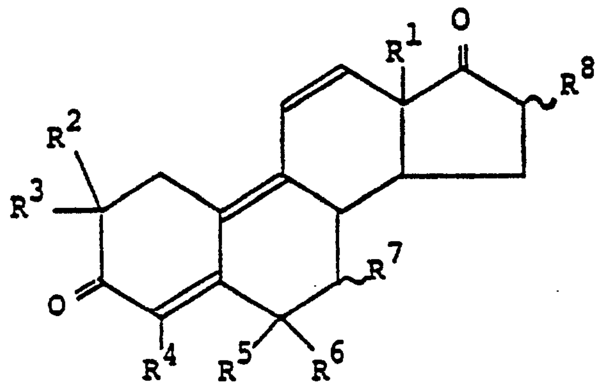

(II)

dans laquelle $R^1$ à $R^8$ sont tels que présentement définis,
avec l'éthylène glycol, le triméthylène glycol ou le diméthyl-2,2 triméthylène glycol, dans des conditions acides, pour obtenir un acétal de formule (III):

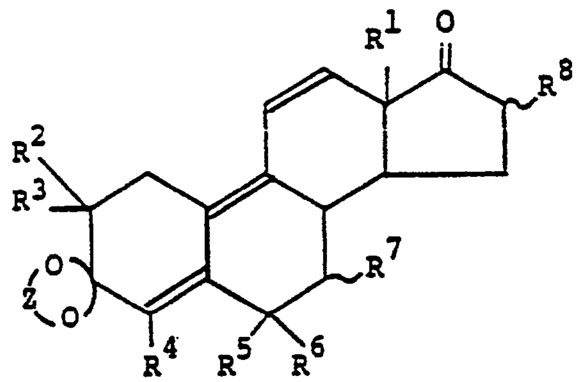

(III)

dans laquelle:
$R^1$ à $R^8$ sont tels que présentement définis; et
Z représente des groupes éthylène, triméthylène ou diméthyl-2,2 triméthylène,
faire réagir l'acétal ainsi obtenu (III) avec le méthylure de diméthylsulfonium ou le méthylure de diméthyl-oxosulfonium, pour obtenir un époxytriène de formule (IV):

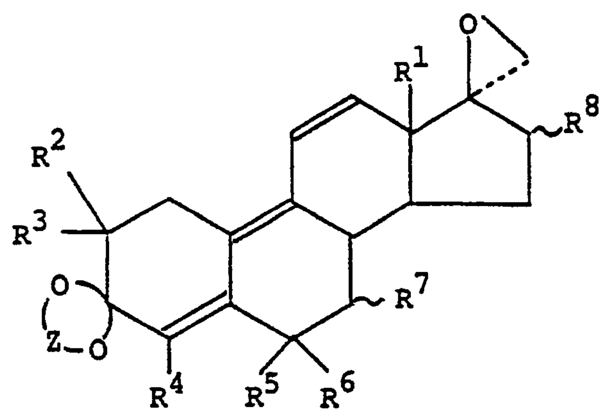

(IV)

dans laquelle:
$R^1$ à $R^8$ et Z sont tels que présentement définis, faire réagir l'époxytriène (IV) ainsi obtenu avec un cyanure, un halogénure, un alcoolate métallique, un alkylsulfure métallique ou un thiocyanate, pour obtenir le composé de formule (V):

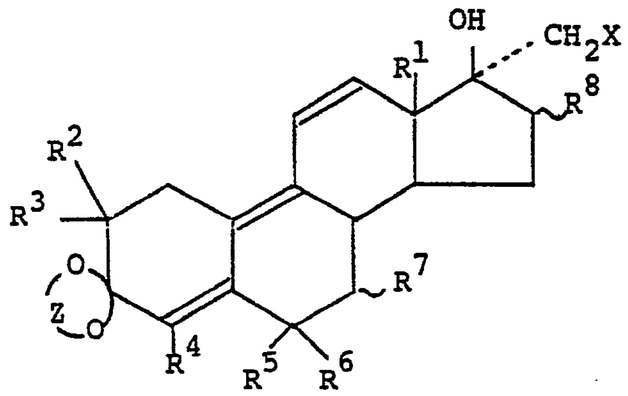

(V)

dans laquelle $R^1$ à $R^8$, X et Z sont tels que présentement définis, traiter le composé (V) ainsi obtenu par un acide, pour obtenir le dérivé de gonatriène de formule (I'):

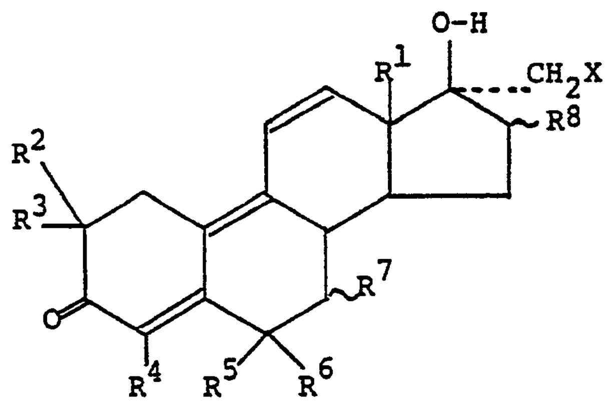

(I')

dans laquelle $R^1$ à $R^8$ et X sont tels que présentement définis, et si nécessaire, soumettre le dérivé de gonatriène (I') à une acylation ou une éthérification.

11. Procédé de préparation d'un dérivé de gonatriène représenté par la formule générale (I):

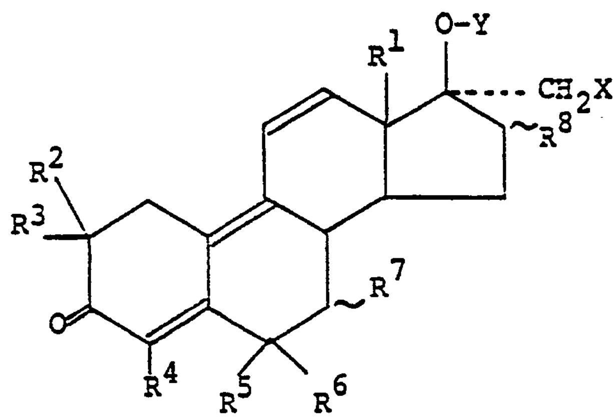

(I)

dans laquelle:
$R^1$ représente un groupe alkyle ayant 1 à 3 atomes de carbone;
X représente un atome d'halogène, un groupe cyano ou –SCN;
$R^2$, $R^3$, $R^4$ et $R^7$ représentent indépendamment un atome d'hydrogène, un atome d'halogène ou un groupe alkyle ayant 1 à 3 atomes de carbone;
$R^5$ et $R^6$ représentent indépendamment un atome d'hydrogène, un atome d'halogène, un groupe alkyle ayant 1 à 3 atomes de carbone ou un groupe hydroxy, ou bien forment conjointement un groupe oxo;
$R^8$ représente un atome d'hydrogène ou un groupe alkyle ayant 1 à 3 atomes de carbone; et
Y représente un atome d'hydrogène, $-COR^{10}$ ou $R^{11}$, où $R^{10}$ et $R^{11}$ représentent chacun un groupe alkyle ou un groupe aryle facultativement substitués par des atomes d'halogène,

à la condition que les groupes définis ci-dessus ne peuvent pas avoir simultanément les significations suivantes:
$R_2$, $R_3$, $R_4$, $R_5$, $R_6$, $R_7$, $R_8$ et Y représentent des atomes d'hydrogène; et
X représente $-OCH_3$,
qui comprend les étapes consistant à:
faire réagir une dicétone de formule (II):

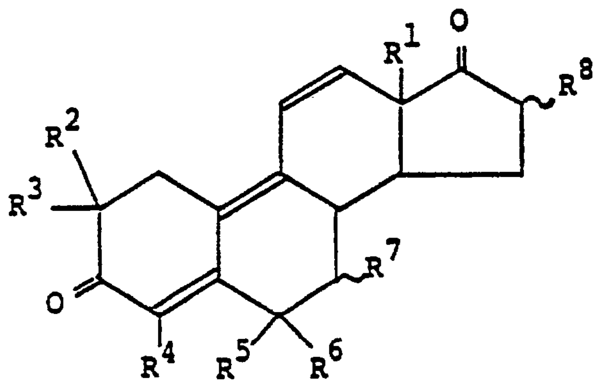

(II)

dans laquelle $R^1$ à $R^8$ sont tels que présentement définis,
avec l'éthylène glycol, le triméthylène glycol ou le diméthyl-2,2 triméthylène glycol, dans des conditions acides, pour obtenir un acétal de formule (III):

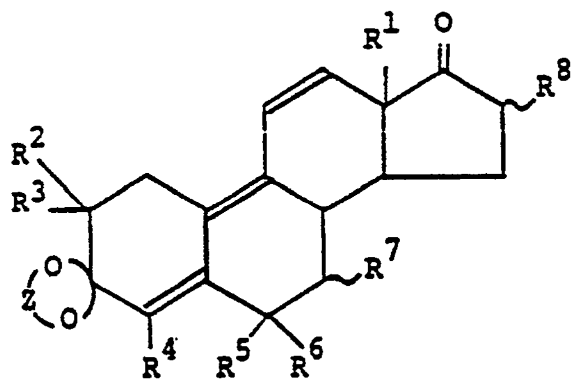

(III)

dans laquelle:
$R^1$ à $R^8$ sont tels que présentement définis; et Z représente des groupes éthylène, triméthylène ou diméthyl-2,2 triméthylène,
faire réagir l'acétal ainsi obtenu (III) avec le méthylure de diméthylsulfonium ou le méthylure de diméthyloxosulfonium, pour obtenir un époxytriène de formule (IV):

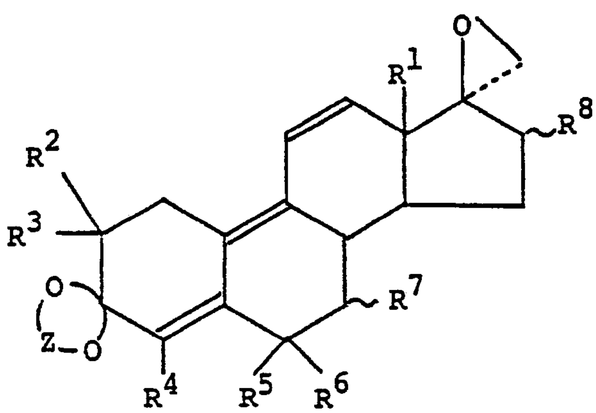

(IV)

dans laquelle:
$R^1$ à $R^8$ et Z sont tels que présentement définis, faire réagir l'époxytriène (IV) ainsi obtenu avec l'acide

cyanique, un halogénure d'hydrogène ou l'acide thiocyanique, pour obtenir le dérivé de gonatriène de formule (I'):

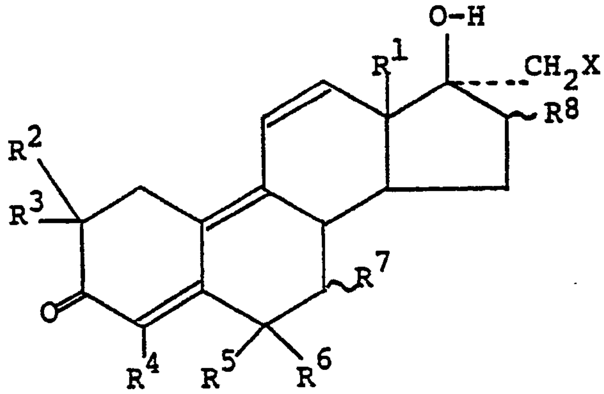

(I')

dans laquelle $R^1$ à $R^8$, X et Z sont tels que présentement définis, et

si nécessaire, soumettre le dérivé de gonatriène (I') ainsi obtenu à une acylation ou une éthérification.

12. Procédé de préparation d'un dérivé de gonatriène représenté par la formule générale (I):

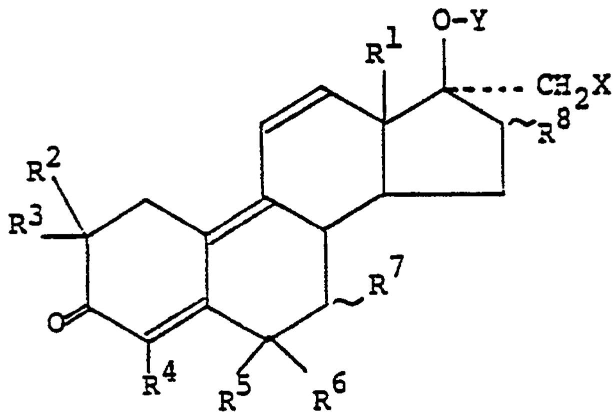

(I)

dans laquelle:

$R^1$ représente un groupe alkyle ayant 1 à 3 atomes de carbone;

X représente un atome d'halogène, un groupe cyano, $-OR^9$, $-SR^9$, $-SOR^9$ ou $-SCN$, où $R^9$ est un groupe alkyle ayant 1 à 3 atomes de carbone;

$R^2$, $R^3$, $R^4$ et $R^7$ représentent indépendamment un atome d'hydrogène, un atome d'halogène ou un groupe alkyle ayant 1 à 3 atomes de carbone;

$R^5$ et $R^6$ représentent indépendamment un atome d'hydrogène, un atome d'halogène, un groupe alkyle ayant 1 à 3 atomes de carbone ou un groupe hydroxy, ou bien forment conjointement un groupe oxo;

$R^8$ représente un atome d'hydrogène ou un groupe alkyle ayant 1 à 3 atomes de carbone; et

Y représente un atome d'hydrogène, $-COR^{10}$ ou $R^{11}$, où $R^{10}$ et $R^{11}$ représentent chacun un groupe alkyle ou un groupe aryle facultativement substitués par des atomes d'halogène,

à la condition que les groupes définis ci-dessus ne peuvent pas avoir simultanément les significations suivantes:

$R_2$, $R_3$, $R_4$, $R_5$, $R_6$, $R_7$, $R_8$ et Y représentent des atomes d'hydrogène; et

X représente $-OCH_3$,

qui comprend les étapes consistant à:

faire réagir un gonadiène de formule (VI):

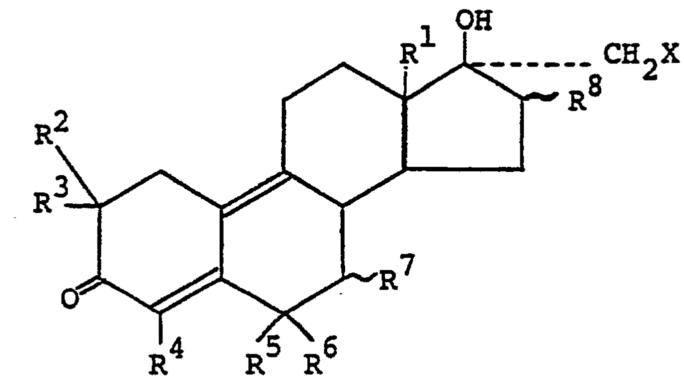

(VI)

dans laquelle $R^1$ à $R^8$ et X sont tels que présentement définis, avec un amine de formule (VII):

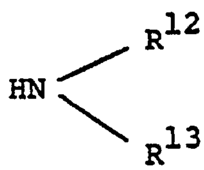

(VII)

dans laquelle $R^{12}$ et $R^{13}$ représentent indépendamment un groupe alkyle ayant 1 à 4 atomes de carbone, ou bien forment conjointement $-(CH_2)_n-$, où n est un nombre entier de 2 à 4, pour obtenir une énamine de formule (VIII):

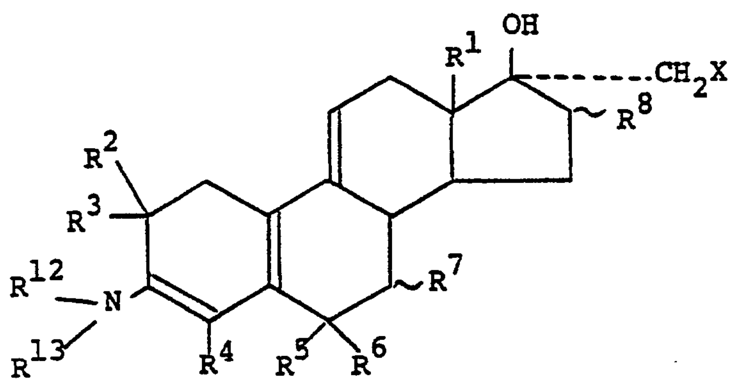

(VIII)

dans laquelle $R^1$ à $R^8$, $R^{12}$, $R^{13}$ et X sont tels que présentement définis, faire réagir l'énamine (VIII) ainsi obtenue avec un acide pour obtenir un diène de formule (IX):

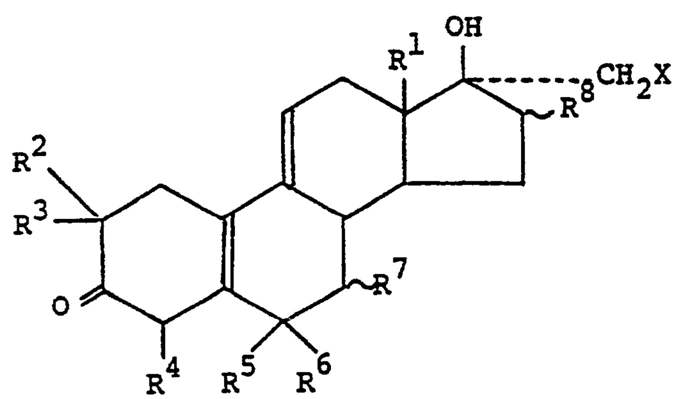

(IX)

dans laquelle $R^1$ à $R^8$ et X sont tels que présentement définis,

faire réagir le diène (IX) ainsi obtenu avec la dichlorodicyanobenzoquinone, pour obtenir le dérivé de gonatriène de formule (I′):

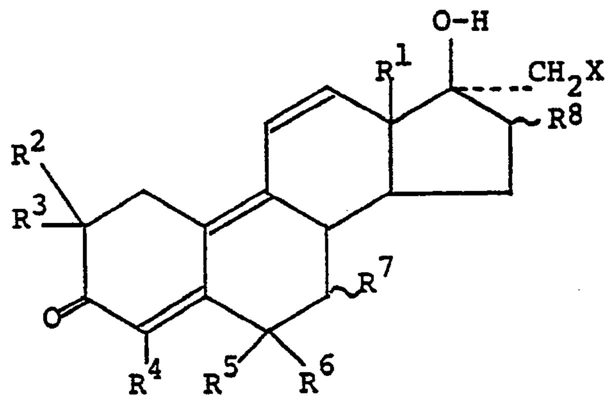

(I')

dans laquelle $R^1$ à $R^8$ et X sont tels que présentement définis et
si nécessaire, soumettre le dérivé de gonatriène (I′) ainsi obtenu à une acylation ou une éthérification.

13. Procédé de préparation d'un dérivé de gonatriène représenté par la formule générale (I):

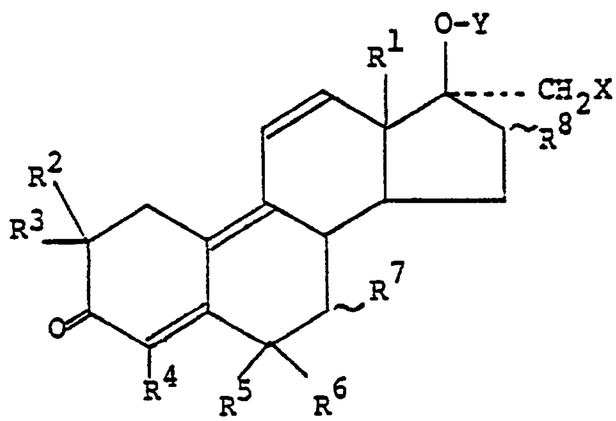

(I)

dans laquelle:
$R^1$ représente un groupe alkyle ayant 1 à 3 atomes de carbone;
X représente $-SOR^9$;
$R^2$, $R^3$, $R^4$ et $R^7$ représentent indépendamment un atome d'hydrogène, un atome d'halogène ou un groupe alkyle ayant 1 à 3 atomes de carbone;
$R^5$ et $R^6$ représentent indépendamment un atome d'hydrogène, un atome d'halogène, un groupe alkyle ayant 1 à 3 atomes de carbone ou un groupe hydroxy, ou bien forment conjointement un groupe oxo;
$R^8$ représente un atome d'hydrogène ou un groupe alkyle ayant 1 à 3 atomes de carbone; et
Y représente un atome d'hydrogène, $-COR^{10}$ ou $R^{11}$, où $R^{10}$ et $R^{11}$ représentent chacun un groupe alkyle ou un groupe aryle facultativement substitués par des atomes d'halogène,
à la condition que les groupes définis ci-dessus ne peuvent pas avoir simultanément les significations suivantes:
$R_2$, $R_3$, $R_4$, $R_5$, $R_6$, $R_7$, $R_8$ et Y représentent des atomes d'hydrogène; et
X représente $-OCH_3$;
qui comprend l'oxydation du dérivé de gonatriène de formule (I″):

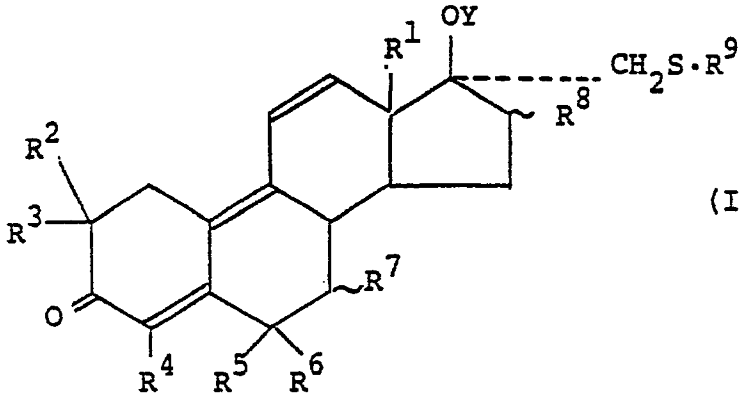

(I")

dans laquelle $R^1$ à $R^9$ et Y sont tels que présentement définis.

14. Agent pharmaceutique pour le traitement de l'endométriose contenant un dérivé de gonatriène représenté par la formule générale (I):

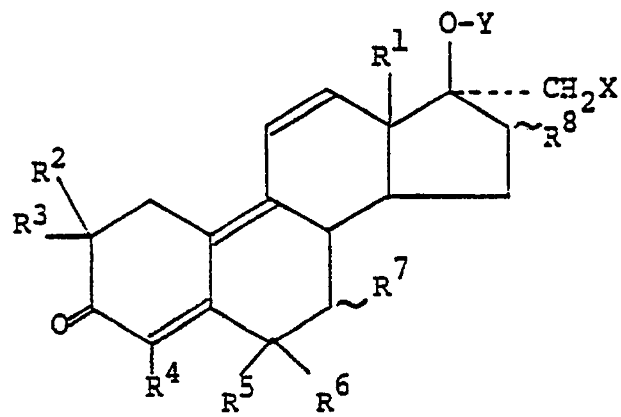

(I)

dans laquelle:
$R^1$ représente un groupe alkyle ayant 1 à 3 atomes de carbone;
X représente un atome d'halogène, un groupe cyano, $-OR^9$, $-SR^9$, $-SOR^9$ ou $-SCN$, où $R^9$ est un groupe alkyle ayant 1 à 3 atomes de carbone;
$R^2$, $R^3$, $R^4$ et $R^7$ représentent indépendamment un atome d'hydrogène, un atome d'halogène ou un groupe alkyle ayant 1 à 3 atomes de carbone;
$R^5$ et $R^6$ représentent indépendamment un atome d'hydrogène, un atome d'halogène, un groupe alkyle ayant 1 à 3 atomes de carbone ou un groupe hydroxy, ou bien forment conjointement un groupe oxo;
$R^8$ représente un atome d'hydrogène ou un groupe alkyle ayant 1 à 3 atomes de carbone; et
Y représente un atome d'hydrogène, $-COR^{10}$ ou $R^{11}$, où $R^{10}$ et $R^{11}$ représentent chacun un groupe alkyle, un groupe aryle, un groupe haloalkyle ou un groupe haloaryle,
à la condition que les groupes définis ci-dessus ne peuvent pas avoir simultanément les significations suivantes:
$R_2$, $R_3$, $R_4$, $R_5$, $R_6$, $R_7$, $R_8$ et Y représentent de atomes d'hydrogène; et
X représente $-OCH_3$.

15. Utilisation d'un dérivé de gonatriène pour la préparation de compositions pharmaceutiques pour le traitement de l'endométriose, ledit dérivé étant représenté par la formule générale (I):

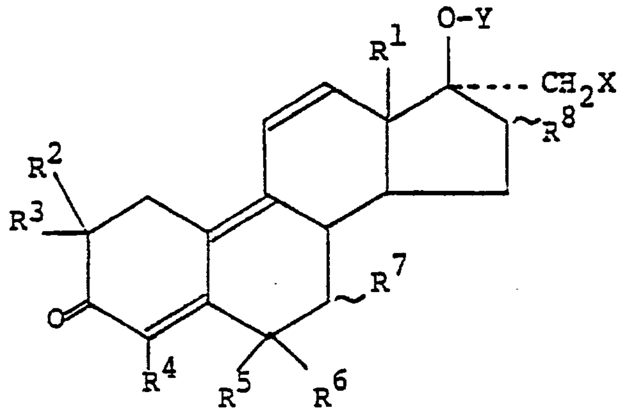

(I)

dans laquelle:
$R^1$ représente un groupe alkyle ayant 1 à 3 atomes de carbone;
X représente un atome d'halogène, un groupe cyano, $-OR^9$, $-SR^9$, $-SOR^9$ ou $-SCN$, où $R^9$ est un groupe alkyle ayant 1 à 3 atomes de carbone;
$R^2$, $R^3$, $R^4$ et $R^7$ représentent indépendamment un atome d'hydrogène, un atome d'halogène ou un groupe alkyle ayant 1 à 3 atomes de carbone;
$R^5$ et $R^6$ représentent indépendamment un atome d'hydrogène, un atome d'halogène, un groupe alkyle ayant 1 à 3 atomes de carbone ou un groupe hydroxy, ou bien forment conjointement un groupe oxo;
$R^8$ représente un atome d'hydrogène ou un groupe alkyle ayant 1 à 3 atomes de carbone; et
Y représente un atome d'hydrogène, $-COR^{10}$ ou $R^{11}$, où $R^{11}$ et $R^{11}$ représentent chacun un groupe alkyle, un groupe aryle, un groupe haloalkyle ou un groupe haloaryle.